(19) Europäisches Patentamt
European Patent Office
Office européen des brevets

(11) Veröffentlichungsnummer: **0 139 613**
**A1**

# (12) EUROPÄISCHE PATENTANMELDUNG

(21) Anmeldenummer: **84810417.0**

(22) Anmeldetag: **23.08.84**

(51) Int. Cl.[4]: **C 07 D 239/47**
C 07 D 239/42, C 07 D 239/46
C 07 D 239/48, C 07 D 239/28
A 01 N 43/54

(30) Priorität: **29.08.83 CH 4723/83**

(43) Veröffentlichungstag der Anmeldung:
**02.05.85 Patentblatt 85/18**

(84) Benannte Vertragsstaaten:
**AT BE CH DE FR GB IT LI LU NL SE**

(71) Anmelder: **CIBA-GEIGY AG**
**Postfach**
**CH-4002 Basel(CH)**

(72) Erfinder: **Hubele, Adolf, Dr.**
**Obere Egg 9**
**CH-4312 Magden(CH)**

(72) Erfinder: **Eckhardt, Wolfgang, Dr.**
**Breslauerstrasse 14**
**D-7850 Lörrach(DE)**

(72) Erfinder: **Sturm, Elmar, Dr.**
**Klusstrasse 66**
**CH-4147 Aesch(CH)**

(72) Erfinder: **Zondler, Helmut, Dr.**
**Oberwilerstrasse 49**
**CH-4103 Bottmingen(CH)**

## (54) N-(2-Nitrophenyl)-4-aminopyrimidin-Derivate, deren Herstellung und Verwendung.

(57) Es werden neue N(2-Nitrophenyl)-4-amino-pyrimidin-Derivate der allgemeinen Formel I beschrieben,

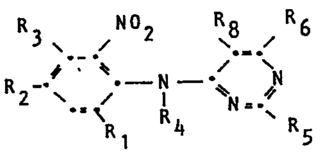

(I)

worin

$R_1$ für $NO_2$ oder $CF_3$ steht;
$R_2$ für $NO_2$ oder $CF_3$ steht;
$R_3$ Wasserstoff oder Halogen bedeutet;
$R_4$ für Wasserstoff oder die Gruppe $-C(O)R_7$ steht; wobei $R_7$ einen unsubstituierten oder substituierten Rest aus der Reihe $C_1$-$C_{12}$-Alkyl, $C_2$-$C_6$-Alkenyl, $C_3$-$C_7$-Cycloalkyl, Phenyl und Herterocyclyl repräsentiert;
$R_5$, $R_6$ und $R_8$ unabhängig voneinander für Halogen, Cyano, Rhodano, Nitro oder eine der Gruppen $-N(R_9)(R_{10})$, $-R_{11}$, $R_{12}$-O- oder $R_{13}$-$S(O)_n$ stehen, wobei $R_9$ und $R_{10}$ unabhängig voneinander Wasserstoff oder einen unsubstituierten oder substituierten Rest aus der Reihe $C_1$-$C_{12}$-Alkyl, $C_3$-$C_6$-Alkenyl und Phenyl darstellen oder zusammen mit dem Stickstoffatom gemeinsam einen gegebenenfalls substituierten heterocyclischen Ring bilden;
n eine der Zahlen 0, 1 oder 2 bedeutet;
$R_{11}$, $R_{12}$ und $R_{13}$ unabhängig voneinander für einen unsubstituierten oder substituierten Rest aus der Reihe $C_1$-$C_{12}$-Alkyl, $C_2$-$C_6$-Alkenyl, $C_3$-$C_6$-Alkinyl, $C_3$-$C_7$-Cycloalkyl, Phenyl oder Heterocyclyl stehen; darüberhinaus $R_5$ für Wasserstoff, $R_6$ für Wasserstoff oder zwei der Reste $R_5$, $R_6$ und $R_8$ für Wasserstoff stehen können.

Es werden ferner methoden zur Herstellung dieser Produkte offenbart sowie agrochemische Mittel, die als Wirkstoff eine dieser Verbindungen enthalten. Ferner wird ihr Einsatz auf dem Agrargebiet bzw. verwandten Gebieten beschrieben.

Croydon Printing Company Ltd.

CIBA-GEIGY AG 5-14567/=

Basel (Schweiz)

N-(2-Nitrophenyl)-4-aminopyrimidin-Derivate, deren Herstellung und Verwendung

Die vorliegende Erfindung betrifft neue N(2-Nitrophenyl)-4-amino-pyrimidin-Derivate der nachstehenden Formel I.

Die Erfindung betrifft ferner die Herstellung dieser Substanzen sowie agrochemische Mittel, die als Wirkstoff mindestens eine dieser Verbindungen enthalten. Die Erfindung betrifft ebenso die Herstellung der genannten Mittel sowie die Verwendung der Wirkstoffe oder der Mittel zur Bekämpfung von schädlichen Mikroorganismen, vorzugsweise pflanzenschädigenden Pilzen.

Bei den erfindungsgemässen Verbindungen handelt es sich um solche der allgemeinen Formel I:

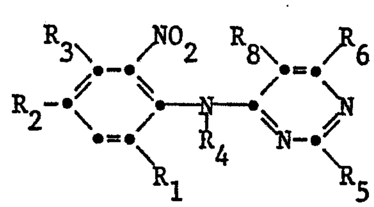

(I)

worin

$R_1$ für $NO_2$ oder $CF_3$ steht;

$R_2$ für $NO_2$ oder $CF_3$ steht;

$R_3$ Wasserstoff oder Halogen bedeutet;

$R_4$ für Wasserstoff oder die Gruppe $-C(O)R_7$ steht; wobei

$R_7$ einen unsubstituierten oder substituierten Rest aus der Reihe $C_1$-$C_{12}$-Alkyl, $C_2$-$C_6$-Alkenyl, $C_3$-$C_7$-Cycloalkyl, Phenyl und Heterocyclyl repräsentiert;

$R_5$, $R_6$ und $R_8$ unabhängig voneinander für Halogen, Cyano, Rhodano, Nitro oder eine der Gruppen $-N(R_9)(R_{10})$, $-R_{11}$, $R_{12}-O-$ oder $R_{13}-S(O)_n$ stehen, wobei $R_9$ und $R_{10}$ unabhängig voneinander Wasserstoff oder einen unsubstituierten oder substituierten Rest aus der Reihe $C_1$-$C_{12}$-Alkyl, $C_3$-$C_6$-Alkenyl und Phenyl darstellen oder zusammen mit dem Stickstoffatom gemeinsam einen gegebenenfalls substituierten heterocyclischen Ring bilden;

n eine der Zahlen 0, 1 oder 2 bedeutet;

$R_{11}$, $R_{12}$ und $R_{13}$ unabhängig voneinander für einen unsubstituierten oder substituierten Rest aus der Reihe $C_1$-$C_{12}$-Alkyl, $C_2$-$C_6$-Alkenyl, $C_3$-$C_6$-Alkinyl, $C_3$-$C_7$-Cycloalkyl, Phenyl oder Heterocyclyl stehen; darüberhinaus $R_5$ für Wasserstoff, $R_6$ für Wasserstoff oder zwei der Reste $R_5$, $R_6$ und $R_8$ für Wasserstoff stehen können.

Unter dem Begriff Alkyl selbst oder als Bestandteil eines anderen Substituenten, wie Alkoxy, Alkylmercapto, Haloalkyl, etc., sind je nach Zahl der angegebenen Kohlenstoffatome beispielsweise die folgenden geradkettigen oder verzweigten Gruppen zu verstehen: Methyl, Ethyl, Propyl, Butyl, Pentyl, Hexyl, Heptyl, Octyl, Nonyl, Decyl, Undecyl, Dodecyl usw. sowie ihre Isomeren, wie z.B. Isopropyl, Isobutyl, tert.-Butyl, Isopentyl usw. Die Vorsilbe Halo in der Bezeichnung eines Substituenten bedeutet hier und im folgenden, dass dieser Substituent einfach bis perhalogeniert auftreten kann. Halogen und Halo stehen stellvertretend für F, Cl, Br oder J. Haloalkyl steht somit für einen einfach bis perhalogenierten Alkylrest, wie z.B. $CHCl_2$, $CH_2F$, $CCl_3$, $CH_2Cl$, $CHF_2$, $CH_2CH_2Br$, $C_2Cl_5$, CHBrCl usw., vorzugsweise für $CF_3$. Alkenyl steht z.B. für Propenyl-(1), Allyl, Butenyl-(1), Butenyl-(2) oder Butenyl-(3) sowie Ketten mit mehreren Doppelbindungen. Cycloalkyl steht je nach Zahl der genannten Kohlenstoffatome z.B. für Cyclopropyl, Cyclobutyl, Cyclopentyl, Cyclohexyl, Cycloheptyl, Cyclooctyl usw. Alkinyl bedeutet z.B.

Propinyl-(2), Propargyl, Butinyl-(1), Butinyl-(2) usw., vorzugsweise Propargyl.

Der Ausdruck "Heterocyclyl" oder "hererocyclischer Ring" soll hier und im folgenden für einen gesättigten oder ungesättigten heterocyclischen Rest mit einem oder mehreren Heteroatomen, vorzugsweise einen gesättigten oder ungesättigten fünf- oder sechsgliedrigen Heterocyclus repräsentieren mit ein bis drei gleichen oder verschiedenen Heteroatomen, wie z.B. Sauerstoff, Stickstoff oder Schwefel. Typische Vertreter derartiger Heterocyclen sind: Tetrahydrofuran, Furan, Tetrahydrothiophen, Thiophen, Pyrrolidin, Pyrrol, Pyrrolin, Pyrazol, Imidazol, Pyrazolin, Oxazol, Thiazol, Isoxazol, Isothiazol, Pyran, Dihydropyran, Tetrahydropyran, Thiopyran, Dihydrothiopyran, Tetrahydrothiopyran, Pyridazin, Dihydropyridazin, Tetrahydropyridazin, Pyrimidin, Dihydropyrimidin, Tetrahydropyrimidin, Pyrazin, Dihydropyrazin, Tetrahydropyrazin, Morpholin, Thiazin, Dihydrothiazin, Tetrahydrothiazin, Piperazin und Triazin.

Die Verbindungen der Formel I sind bei Raumtemperatur Oele, Harze oder überwiegend kristalline Feststoffe, die sich durch sehr wertvolle pestizide Eigenschaften auszeichnen. Sie lassen sich beispielsweise auf dem Agrarsektor oder verwandten Gebieten präventiv und kurativ zur Bekämpfung phytopathogener Schädlinge, wie z.B. pflanzenpathogenen Pilzen einsetzen. Die erfindungsgemässen Wirkstoffe der Formel I zeichnen sich in weiten Anwendungskonzentrationen durch eine hohe pestizide Aktivität und grosse Wirkungsbreite aus und können problemlos, insbesondere im Agrarbereich eingesetzt werden.

Folgende Wirkstoffgruppen sind auf Grund ihrer ausgeprägten pestiziden, insbesondere pflanzenfungiziden Aktivität bevorzugt:

<u>Gruppe Ia:</u> Verbindungen der Formel I, worin $R_1$ für $NO_2$ oder $CF_3$ steht; $R_2$ für $NO_2$ oder $CF_3$ steht; $R_3$ Wasserstoff oder Halogen bedeutet; $R_4$ für Wasserstoff oder die Gruppe $-C(O)R_7$ steht; wobei $R_7$ unsubsti-

tuiertes oder durch Halogen, $C_1$-$C_3$-Alkoxy oder $C_1$-$C_3$-Alkylthio substituiertes $C_1$-$C_4$-Alkyl, unsubstituiertes oder durch Halogen substituiertes $C_2$-$C_4$-Alkenyl, $C_3$-$C_6$-Cycloalkyl, unsubstituiertes oder durch Halogen, Nitro oder $C_1$-$C_3$-Alkyl substituiertes Phenyl oder einen unsubstituierten oder durch Halogen, $C_1$-$C_3$-Alkyl oder Nitro substituierten, ungesättigten oder gesättigten fünf- bis sechsgliedrigen heterocyclischen Ring mit ein bis drei gleichen oder verschiedenen Heteroatomen repräsentiert; $R_5$, $R_6$ und $R_8$ unabhängig voneinander für Halogen, Cyano, Rhodano, Nitro oder eine der Gruppen $-N(R_9)(R_{10})$, $-R_{11}$, $R_{12}$-O- oder $R_{13}$-$S(O)_n$ stehen, wobei $R_9$ und $R_{10}$ unabhängig voneinander für Wasserstoff, unsubstituiertes oder durch Hydroxy oder $C_1$-$C_4$-Alkoxy substituiertes $C_1$-$C_{12}$-Alkyl, $C_3$-$C_6$-Alkenyl, unsubstituiertes oder durch Halogen, Nitro, $C_1$-$C_4$-Alkyl oder $C_1$-$C_4$-Alkoxy substituiertes Phenyl stehen oder zusammen mit dem Stickstoffatom gemeinsam einen ungesättigten oder gesättigten, unsubstituierten oder durch Halogen, $C_1$-$C_4$-Alkyl oder $C_1$-$C_4$-Alkoxy substituierten, fünf- bis sechsgliedrigen heterocyclischen Ring bilden, der noch ein oder zwei zusätzliche Heteroatome enthalten kann; n eine der Zahlen 0, 1 oder 2 bedeutet; $R_{11}$, $R_{12}$ und $R_{13}$ unabhängig voneinander für unsubstituiertes oder durch Halogen substituiertes $C_2$-$C_6$-Alkenyl; unsubstituiertes oder durch Halogen, Nitro, Cyano, Phenyl, Phenoxy, Phenylthio oder durch einen gesättigten oder ungesättigten, fünf- bis sechsgliedrigen heterocyclischen Ring mit ein bis drei Heteroatomen substituiertes $C_1$-$C_{12}$-Alkyl, wobei jeder cyclische Rest seinerseits unsubstituiert oder durch Halogen, Nitro, $C_1$-$C_4$-Alkyl, $C_1$-$C_4$-Haloalkyl oder $C_1$-$C_4$-Alkoxy substituiert ist; unsubstituiertes oder durch Halogen substituiertes $C_3$-$C_6$-Alkinyl; unsubstituiertes oder durch Halogen oder $C_1$-$C_3$-Alkyl substituiertes $C_3$-$C_7$-Cycloalkyl, unsubstituiertes oder durch Halogen, $C_1$-$C_4$-Haloalkyl, $C_1$-$C_4$-Alkyl oder $C_1$-$C_4$-Alkoxy substituiertes Phenyl oder für einen ungesättigten oder gesättigten, fünf- bis sechsgliedrigen, unsubstituierten oder durch Halogen, Nitro, $C_1$-$C_4$-Alkyl, $C_1$-$C_4$-Haloalkyl oder $C_1$-$C_4$-Alkoxy substituierten heterocyclischen Ring mit ein bis drei Heteroatomen stehen; und darüberhinaus $R_5$ für Wasserstoff, $R_6$ für Wasserstoff oder zwei der Reste $R_5$, $R_6$ und $R_8$ für Wasserstoff stehen können.

Gruppe Ib: Verbindungen der Formel I, worin $R_1$ für $NO_2$ oder $CF_3$ steht; $R_2$ für $NO_2$ oder $CF_3$ steht; $R_3$ Wasserstoff oder Halogen bedeutet; $R_4$ für Wasserstoff steht; $R_5$, $R_6$ und $R_8$ unabhängig voneinander für Halogen, Cyano, Rhodano, Nitro oder eine der Gruppen $-N(R_9)(R_{10})$, $-R_{11}$, $R_{12}-O-$ oder $R_{13}-S(O)_n$ stehen, wobei $R_9$ und $R_{10}$ unabhängig voneinander für Wasserstoff, unsubstituiertes oder durch Hydroxy oder $C_1$-$C_4$-Alkoxy substituiertes $C_1$-$C_{12}$-Alkyl, $C_3$-$C_6$-Alkenyl, unsubstituiertes oder durch Halogen, Nitro, $C_1$-$C_4$-Alkyl oder $C_1$-$C_4$-Alkoxy substituiertes Phenyl stehen oder zusammen mit dem Stickstoffatom gemeinsam einen ungesättigten oder gesättigen, unsubstituierten oder durch Halogen, $C_1$-$C_4$-Alkyl oder $C_1$-$C_4$-Alkoxy substituierten, fünf- bis sechsgliedrigen heterocyclischen Ring bilden, der noch ein oder zwei zusätzliche Heteroatome enthalten kann; n eine der Zahlen 0, 1 oder 2 bedeutet; $R_{11}$, $R_{12}$ und $R_{13}$ unabhängig voneinander für unsubstituiertes oder durch Halogen substituiertes $C_2$-$C_6$-Alkenyl; unsubstituiertes oder durch Halogen, Nitro, Cyano, Phenyl, Phenoxy, Phenylthio oder durch einen gesättigten oder ungesättigten, fünf- bis sechsgliedrigen heterocyclischen Ring mit ein bis drei Heteroatomen substituiertes $C_1$-$C_{12}$-Alkyl, wobei jeder cyclische Rest seinerseits unsubstituiert oder durch Halogen, Nitro, $C_1$-$C_4$-Alkyl, $C_1$-$C_4$-Haloalkyl oder $C_1$-$C_4$-Alkoxy substituiert ist; unsubstituiertes oder durch Halogen substituiertes $C_3$-$C_6$-Alkinyl; unsubstituiertes oder durch Halogen oder $C_1$-$C_3$-Alkyl substituiertes $C_3$-$C_7$-Cycloalkyl, unsubstituiertes oder durch Halogen, $C_1$-$C_4$-Haloalkyl, $C_1$-$C_4$-Alkyl oder $C_1$-$C_4$-Alkoxy substituiertes Phenyl oder für einen ungesättigten oder gesättigten, fünf- bis sechsgliedrigen, unsubstituierten oder durch Halogen, Nitro, $C_1$-$C_4$-Alkyl, $C_1$-$C_4$-Haloalkyl oder $C_1$-$C_4$-Alkoxy substituierten heterocyclischen Ring mit ein bis drei Heteroatomen stehen; und darüberhinaus $R_5$ für Wasserstoff, $R_6$ für Wasserstoff oder zwei der Reste $R_5$, $R_6$ und $R_8$ für Wasserstoff stehen können.

Gruppe Ic: Verbindungen der Formel I, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_5$ für Halogen, Cyano, Rhodano, Nitro oder eine der Gruppen $-N(R_9)(R_{10})$, $-R_{11}$, $R_{12}-O-$ oder $R_{13}-S(O)_n$ steht, wobei $R_9$ und $R_{10}$ unabhängig voneinander für Wasserstoff, $C_1$-$C_4$-Alkyl, $C_3$-$C_4$-Alkenyl, unsubstituiertes oder durch Halogen, Nitro, Methyl oder Methoxy substituiertes Phenyl stehen oder zusammen einen unsubstituierten oder durch Methyl substituierten Piperidinring bilden; n für die Zahl 2 steht; $R_{11}$, $R_{12}$ und $R_{13}$ unabhängig voneinander für $C_3$-$C_4$-Alkenyl, unsubstituierts oder durch Halogen, Nitro, Cyano, Phenyl, Phenoxy, Phenylthio oder durch einen ungesättigten oder gesättigten, fünf- oder sechsgliedrigen heterocyclischen Ring mit ein oder zwei Heteroatomen aus der Reihe Stickstoff, Sauerstoff und Schwefel substituiertes $C_1$-$C_{12}$-Alkyl, wobei jeder heterocyclische Rest seinerseits unsubstituiert oder durch Halogen, Nitro, Methyl, $CF_3$ oder Methoxy substituiert ist; für $C_3$-$C_4$-Alkinyl, $C_3$-$C_6$-Cycloalkyl, unsubstituiertes oder durch Halogen, Nitro, $C_1$-$C_2$-Alkyl, $CF_3$ oder $C_1$-$C_2$-Alkoxy substituiertes Phenyl stehen; und $R_6$ und $R_8$ Wasserstoff bedeuten.

Gruppe Id: Verbindungen der Formel I, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_5$ für Wasserstoff steht; $R_6$ für Wasserstoff, Halogen, Cyano, Rhodano, Nitro oder eine der Gruppen $-N(R_9)(R_{10})$, $-R_{11}$, $R_{12}-O-$ oder $R_{13}-S(O)_n$ steht, wobei $R_9$ und $R_{10}$ unabhängig voneinander für Wasserstoff, $C_1$-$C_3$-Alkyl oder zusammen mit dem Stickstoffatom für Piperidin stehen; n für die Zahl 2 steht; $R_{11}$, $R_{12}$ und $R_{13}$ unabhängig voneinander für $C_3$-$C_4$-Alkenyl, unsubstituiertes oder durch Halogen, Nitro, Cyano, Phenyl, Phenoxy oder Phenylthio substituiertes $C_1$-$C_4$-Alkyl, $C_3$-$C_6$-Cycloalkyl oder unsubstituiertes

oder durch Halogen, $CF_3$, $C_1$-$C_2$-Alkyl oder $C_1$-$C_2$-Alkoxy substituiertes Phenyl stehen; und $R_8$ Wasserstoff bedeutet.

Gruppe Ie: Verbindungen der Formel I, worin $R_1$ für $NO_2$ oder $CF_3$ steht; $R_2$ für $NO_2$ oder $CF_3$ steht; $R_3$ Wasserstoff oder Chlor bedeutet; $R_4$, $R_5$ und $R_6$ für Wasserstoff stehen; und $R_8$ Halogen, Cyano, Rhodano, Nitro oder eine der Gruppen $-N(R_9)(R_{10})$, $-R_{11}$, $R_{12}$-O- oder $R_{13}$-$S(O)_n$ repräsentiert, wobei $R_9$ und $R_{10}$ unabhängig voneinander für Wasserstoff, $C_1$-$C_3$-Alkyl oder zusammen mit dem Stickstoffatom für Piperidin stehen; n für die Zahl 2 steht; und $R_{11}$, $R_{12}$ und $R_{13}$ unabhängig voneinander für $C_3$-$C_4$-Alkenyl, unsubstituiertes oder durch Halogen, Nitro, Cyano, Phenyl, Phenoxy oder Phenylthio substituiertes $C_1$-$C_4$-Alkyl, $C_3$-$C_6$-Cycloalkyl oder unsubstituiertes oder durch Halogen, $CF_3$, $C_1$-$C_2$-Alkyl oder $C_1$-$C_2$-Alkoxy substituiertes Phenyl stehen.

Gruppe If: Verbindungen der Formel I, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_5$ und $R_8$ unabhängig voneinander für Halogen, Cyano, Rhodano, Nitro oder eine der Gruppen $-N(R_9)(R_{10})$, $-R_{11}$, $R_{12}$-O- oder $R_{13}$-$S(O)_n$ steht, wobei $R_9$ und $R_{10}$ unabhängig voneinander für Wasserstoff, $C_1$-$C_4$-Alkyl, $C_3$-$C_4$-Alkenyl , unsubstituiertes oder durch Halogen, Nitro, Methyl oder Methoxy substituiertes Phenyl stehen oder zusammen einen unsubstituierten oder durch Methyl substituierten Ring ausgewählt aus der Reihe Piperidin, Tetrahydrofuryl und Morpholin bilden; n für die Zahl 2 steht; $R_{11}$, $R_{12}$ und $R_{13}$ unabhängig voneinander für $C_3$-$C_4$-Alkenyl, unsubstituiertes oder durch Halogen, Nitro, Cyano, Phenyl, Phenoxy, Phenylthio oder durch einen ungesättigten oder gesättigten, fünf- oder sechsgliedrigen heterocyclischen Ring mit ein oder zwei Heteroatomen aus der Reihe Stickstoff, Sauerstoff und Schwefel substituiertes $C_1$-$C_{12}$-Alkyl, wobei jeder heterocyclische

Rest seinerseits unsubstituiert oder durch Halogen, Nitro, Methyl, $CF_3$ oder Methoxy substituiert ist; für $C_3$-$C_4$-Alkinyl, $C_3$-$C_6$-Cycloalkyl, unsubstituiertes oder durch Halogen, Nitro, $C_1$-$C_2$-Alkyl, $CF_3$ oder $C_1$-$C_2$-Alkoxy substituiertes Phenyl stehen; und $R_6$ Wasserstoff bedeutet.

Gruppe Ig: Verbindungen der Formel I, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_6$ und $R_8$ unabhängig voneinander für Halogen, Cyano, Rhodano, Nitro oder eine der Gruppen $-N(R_9)(R_{10})$, $-R_{11}$, $R_{12}$-O- oder $R_{13}$-$S(O)_n$ steht, wobei $R_9$ und $R_{10}$ unabhängig voneinander für Wasserstoff, $C_1$-$C_4$-Alkyl, $C_3$-$C_4$-Alkenyl, unsubstituiertes oder durch Halogen, Nitro, Methyl oder Methoxy substituiertes Phenyl stehen oder zusammen einen unsubstituierten oder durch Methyl substituierten Piperidinring bilden; n für die Zahl 2 steht; $R_{11}$, $R_{12}$ und $R_{13}$ unabhängig voneinander für $C_3$-$C_4$-Alkenyl, unsubstituiertes oder durch Halogen, Nitro, Cyano, Phenyl, Phenoxy, Phenylthio oder durch einen ungesättigten oder gesättigten, fünf- oder sechsgliedrigen heterocyclischen Ring mit ein oder zwei Heteroatomen aus der Reihe Stickstoff, Sauerstoff und Schwefel substituiertes $C_1$-$C_4$-Alkyl, wobei jeder heterocyclische Rest seinerseits unsubstituiert oder durch Halogen, Nitro, Methyl, $CF_3$ oder Methoxy substituiert ist; für $C_3$-$C_4$-Alkinyl, $C_3$-$C_6$-Cycloalkyl, unsubstituiertes oder durch Halogen, Nitro, $C_1$-$C_2$-Alkyl, $CF_3$ oder $C_1$-$C_2$-Alkoxy substituiertes Phenyl stehen; und $R_5$ Wasserstoff bedeutet.

Besonders bevorzugte Einzelsubstanzen sind z.B.:

N-(3'-Chlor-2',6'-dinitro-4'-trifluormethylphenyl)-4-amino-6-chlor-pyrimidin (Nr. 2.2);

N-(3'-Chlor-2',6'-dinitro-4'-trifluormethylphenyl)-4-amino-6-methylmercapto-pyrimidin (Nr. 2.5);

N-(3'-Chlor-2',6'-dinitro-4'-trifluormethylphenyl)-4-amino-6-methoxy-pyrimidin (Nr. 2.4);

N-(3'-Chlor-2',6'-dinitro-4'-trifluormethylphenyl)-4-amino-6-(2,2,2-trifluorethoxy)-pyrimidin (Nr. 2.6);

N-(3'-Chlor-2',6'-dinitro-4'-trifluormethylphenyl)-4-amino-2-methoxy-5-chlor-pyrimidin (Nr. 5.1);

N-(3'-Chlor-2',6'-dinitro-4'-trifluormethylphenyl)-4-amino-2,5-dichlorpyrimidin (Nr. 5.3);

N-(3'-Chlor-2',6'-dinitro-4'-trifluormethylphenyl)-4-amino-2-chlor-5-fluor-pyrimidin (Nr. 5.4);

N-(3'-Chlor-2',6'-dinitro-4'-trifluormethylphenyl)-4-amino-2-methoxy-5-fluor-pyrimidin (Nr. 5.6);

N-(3'-Chlor-2',6'-dinitro-4'-trifluormethylphenyl)-4-amino-5-nitro-6-methoxy-pyrimidin (Nr. 6.1);

N-(3'-Chlor-2',6'-dinitro-4'-trifluormethylphenyl)-4-amino-5-nitro-6-amino-pyrimidin (Nr. 6.2);

N-(3'-Chlor-2',6'-dinitro-4'-trifluormethylphenyl)-4-amino-2-methylmercapto-5-nitro-6-methoxy-pyrimidin (Nr. 7.1);

N-(3'-Chlor-2',6'-dinitro-4'-trifluormethylphenyl)-4-amino-2-methyl-5-ethyl-6-chlor-pyrimidin (Nr. 7.3);.

Die Verbindungen der Formel I werden erfindungsgemäss dadurch hergestellt, dass man eine Verbindung der Formel II

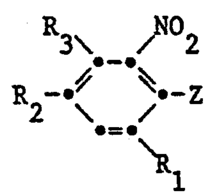

(II)

in Gegenwart einer Base mit einem Pyrimidin-Derivat der Formel III

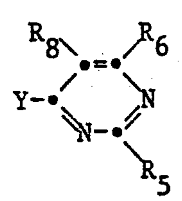

(III)

zu einer Verbindung der Formel I'

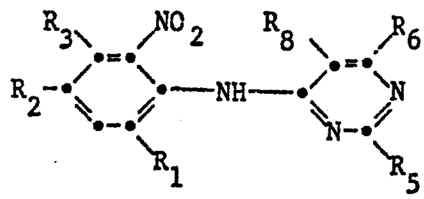

(I')

umsetzt, und letztere zur Herstellung N-acylierter Derivate mit einem reaktionsfähigen Derivat der Carbonsäure IV

$R_4COOH$ (IV)

N-acyliert, wobei die Substituenten $R_1$ bis $R_8$ die unter Formel I angegebenen Bedeutungen haben und Z und Y für $NH_2$ oder Halogen stehen, wobei in dem Fall, in dem Z für Halogen steht, Y $NH_2$ bedeutet und in dem Fall in dem Z für $NH_2$ steht, Y Halogen bedeutet.

Für die Herstellung der Verbindungen der Formel I bzw. I' sind folgende Reaktionsbedingungen vorteilhaft:

Die N-Alkylierung von (II) mit (III) zu (I'), sowie die N-Acylierung von (I') mit (IV) zu (I) erfolgen unter Halogenwasserstoffabspaltung. Die Reaktionstemperaturen liegen bei der N-Alkylierung zwischen -20° und 150°C, vorzugsweise -20° und +30°C, bei der N-Acylierung zwischen 0° und +180°C, vorzugsweise 0° und 150°C bzw. am Siedepunkt des Lösungsmittels bzw. Lösungsmittelgemisches. In beiden Fällen ist die Verwendung von säurebindenden Mitteln bzw.

Kondensationsmitteln vorteilhaft. Als solche kommen organische und anorganische Basen in Betracht, z.B. tertiäre Amine wie Trialkylamine (Trimethylamin, Triethylamin, Tripropylamin usw.), Pyridin und Pyridinbasen (4-Dimethylaminopyridin, 4-Pyrrolidylaminopyridin usw.), Oxide und Hydroxide, Carbonate und Hydrogencarbonate von Alkali- und Erdalkalimetallen sowie Alkaliacetate.

Die Reaktionen können in Anwesenheit von reaktionsinerten Lösungs- oder Verdünnungsmitteln durchgeführt werden. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff,Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Pripionitril; N,N-dialkylierte Amide wie Dimethylformamid; Dimethylsulfoxid; Ketone wie Aceton, Diethylketon, Methylethylketon und Gemische solcher Lösungsmittel untereinander. In manchen Fällen kann das Acylierungs- oder Alkylierungsmittel selbst als Lösungsmittel dienen.

Die Reaktion (II) und (III) kann auch in einem wässrigen Zweiphasensystem nach dem allgemein bekannten Prinzip der Phasentransferkatalyse durchgeführt werden.

Für die organische, mit Wasser nicht mischbare Phase kommen dabei z.B. folgende Lösungsmittel in Frage: Aliphatische und aromatische Kohlenwasserstoffe, wie Pentan, Hexan, Cyclohexan, Petrolether, Ligroin, Benzol, Toluol, Xylole usw., halogenierte Kohlenwasserstoffe, wie Dichlormethan, Chloroform, Tetrachlorkohlenstoff, Ethylendichlorid, 1,2-Dichlorethan, Tetrachlorethylen usw. oder aliphatische Ether, wie Diethylether, Diisopropylether, t-Butylmethylether usw.. Beispiele geeigneter Phasentransfer-Katalysatoren sind: Tetraalkylammoniumhalogenide, -hydrogensulfate oder -hydroxide wie Tetrabutylammoniumchlorid, -bromid, -jodid; Triethylbenzylammoniumchlorid, -bromid; Tetrapropylammoniumchlorid, -bromid, -jodid; usw.. Als Phasentransfer-Katalysatoren kommen auch Phosphonium-Salze in Betracht. Die Reaktionstemperaturen liegen im allgemeinen zwischen -30° und 130°C, bzw. am Siedepunkt des Lösungsmittels oder Lösungsmittelgemisches.

Im übrigen kann bei der Herstellung aller hierin genannten Ausgang-, Zwischen- und Endprodukte können grundsätzlich, sofern nicht ausdrücklich im einzelnen spezifiziert, ein oder mehrere reaktionsinerte Lösungs- oder Verdünnungsmittel anwesend sein. In Frage kommen beispielsweise aliphatische und aromatische Kohlenwasserstoffe wie Benzol, Toluol, Xylole, Petrolether; halogenierte Kohlenwasserstoffe wie Chlorbenzol, Methylenchlorid, Ethylenchlorid, Chloroform, Tetrachlorkohlenstoff, Tetrachlorethylen; Ether und etherartige Verbindungen wie Dialkylether (Diethylether, Diisopropylether, tert.-Butylmethylether usw.), Anisol, Dioxan, Tetrahydrofuran; Nitrile wie Acetonitril, Propionitril; N,N-dialkylierte Amide wie Dimethylformamid; Dimethylsulfoxid; Ketone wie Aceton, Diethylketon, Methylethylketon und Gemische solcher Lösungsmittel untereinander. In

manchen Fällen kann es auch von Vorteil sein, wenn die Reaktion oder Teilschritte einer Reaktion unter Schutzgasatmosphäre und/oder absoluten Lösungsmitteln durchgeführt werden. Als Schutzgase eignen sich inerte Gase wie Stickstoff, Helium, Argon, usw..

Das beschriebene Herstellungsverfahren ist, einschliesslich aller Teilschritte, ein wichtiger Bestandteil der vorliegenden Erfindung.

Es wurde überraschend festgestellt, dass Verbindungen der Formel I ein für praktische Bedürfnisse sehr günstiges biozides Spektrum gegen schädliche Mikroorganismen, insbesondere gegen phytopathogene Pilze aufweisen. Sie besitzen sehr vorteilhafte kurative, systemische und insbesondere präventive Eigenschaften und lassen sich zum Schutz von zahlreichen Kulturpflanzen einsetzen. Mit den Wirkstoffen der Formel I können an Pflanzen oder an Pflanzenteilen (Früchte, Blüten, Laubwerk, Stengel, Knollen, Wurzeln) von unterschiedlichen Nutzkulturen die auftretenden Schädlinge eingedämmt oder vernichtet werden, wobei auch später zuwachsende Pflanzenteile vor phytopathogenen Mikroorganismen verschont bleiben.

Als Mikrobizide sind die Wirkstoffe der Formel I z.B. gegen die den folgenden Klassen angehörenden phytopathogenen Pilze wirksam: Fungi imperfecti (z.B. Botrytis, Helminthosporium, Fusarium, Septoria, Cercospora und Alternaria); Basidiomyceten (z.B. die Gattungen Hemileia, Rhizocotonia, Puccinia); insbesondere wirken sie gegen die Klasse der Ascomyceten (z.B. Venturia, Podosphaera, Erysiphe, Monilinia, Uncinula). Ueberdies wirken die Verbindungen der Formel I systemisch. Sie können ferner als Beizmittel zur Behandlung von Saatgut (Früchte, Knollen, Körner) und Pflanzenstecklingen zum Schutz von Pilzinfektionen sowie gegen im Erdboden auftretende phytopathogene Pilze eingesetzt werden.

Die Erfindung betrifft somit auch mikrobizide Schädlingsbekämpfungsmittel sowie deren Verwendung auf den Agrarsektor oder verwandten Gebieten zur Bekämpfung von pathogenen Mikroorganismen, wie z.B. Pilzen.

Darüberhinaus schliesst die vorliegende Erfindung auch die Herstellung dieser Mittel ein, die gekennzeichnet ist durch das innige Vermischen der Aktivsubstanz mit einem oder mehreren hierin beschriebenen Substanzen bzw. Substanzgruppen. Eingeschlossen ist auch ein Verfahren zur Behandlung von Pflanzen, das sich durch Applikation der Verbindungen der Formel I bzw. der neuen Mittel auszeichnet.

Als Zielkulturen für den hierin offenbarten mikrobiziden Einsatz gelten im Rahmen dieser Erfindung beispielsweise folgende Pflanzenarten: Getreide; (Weizen, Gerste, Roggen, Hafer, Reis, Sorghum und Verwandte); Rüben: (Zucker- und Futterrüben); Kern-, Stein- und Beerenobst: (Aepfel, Birnen, Pflaumen, Pfirsiche, Mandeln, Kirschen, Erd-, Him- und Brombeeren); Hülsenfrüchte: (Bohnen, Linsen, Erbsen, Soja); Oelkulturen: (Raps, Senf, Mohn, Oliven, Sonnenblumen, Kokos, Rizinus, Kakao, Erdnüsse); Gurkengewächse: (Kürbis, Gurken, Melonen); Fasergewächse: (Baumwolle, Flachs, Hanf, Jute); Citrusfrüchte: (Orangen, Zitronen, Pampelmusen, Mandarinen); Gemüsesorten: (Spinat, Kopfsalat, Spargel, Kohlarten, Möhren, Zwiebeln, Tomaten, Kartoffeln, Paprika); Lorbeergewächse: (Avocado, Cinnamonum, Kampfer) oder Pflanzen wie Mais, Tabak, Nüsse, Kaffee, Zuckerrohr, Tee, Weinreben, Hopfen, Bananen- und Naturkautschukgewächse sowie Zierpflanzen (Compositen).

Wirkstoffe der Formel I werden üblicherweise in Form von Zusammensetzungen verwendet und können gleichzeitig oder nacheinander mit weiteren Wirkstoffen auf die zu behandelnde Fläche oder Pflanze gegeben werden. Diese weiteren Wirkstoffe können sowohl Düngemittel, Spurenelement-Vermittler oder andere das Pflanzenwachstum beeinflussende Präparate sein. Es können aber auch selektive Herbizide,

Insektizide, Fungizide, Bakterizide, Nematizide, Molluskizide oder Gemische mehrerer dieser Präparate sein, zusammen mit gegebenenfalls weiteren in der Formulierungstechnik üblichen Trägerstoffen, Tensiden oder anderen applikationsfördernden Zusätzen.

Geeigente Träger und Zusätze können fest oder flüssig sein und entsprechen den in der Formulierungstechnik zweckdienlichen Stoffen, wie z.B. natürlichen oder regenerierten mineralischen Stoffen, Lösungs-, Dispergier-, Netz-, Haft-, Verdickungs-, Binde- oder Düngemitteln.

Ein bevorzugtes Verfahren zum Aufbringen eines Wirkstoffes der Formel I bzw. eines agrochemischen Mittels, das mindestens einen dieser Wirkstoffe enthält, ist das Aufbringen auf das Blattwerk (Blattapplikation). Anzahl der Applikationen und Aufwandmenge richten sich dabei nach dem Befallsdruck für den entsprechenden Erreger (Pilzsorte). Die Wirkstoffe der Formel I können aber auch über den Erdboden durch das Wurzelwerk in die Pflanze gelangen (systemische Wirkung), indem man den Standort der Pflanze mit einer flüssigen Zubereitung tränkt oder die Substanzen in fester Form in den Boden einbringt, z.B. in Form von Granulat (Bodenapplikation). Die Verbindungen der Formel I können aber auch auf Samenkörner aufgebracht werden (Coating), indem man die Körner entweder mit einer flüssigen Zubereitung des Wirkstoffs tränkt oder sie mit einer festen Zubereitung beschichtet. Darüberhinaus sind in besonderen Fällen weitere Applikationsarten möglich, so z.B. die gezielte Behandlung der Pflanzenstengel oder der Knospen.

Die Verbindungen der Formel I werden dabei in unveränderter Form oder vorzugsweise zusammen mit den in der Formulierungstechnik üblichen Hilfsmittel eingesetzt und werden daher z.B. zu Emulsionskonzentraten, streichfähigen Pasten, direkt versprühbaren oder verdünnbaren Lösungen, verdünnten Emulsionen, Spritzpulvern, löslichen Pulvern, Stäubemitteln, Granulaten, durch Verkapselungen in z.B. polymeren Stoffen in bekannter Weise verarbeitet. Die Anwendungsverfahren wie Versprühen,

Vernebeln, Verstäuben, Verstreuen, Bestreichen oder Giessen werden gleich wie die Art der Mittel den angestrebten Zielen und den gegebenen Verhältnissen entsprechend gewählt. Günstige Aufwandmengen liegen im allgemeinen bei 50 g bis 5 kg Aktivsubstanz (AS) je ha; bevorzugt 100 g bis 2 kg AS/ha, insbesondere bei 200 g bis 600 g AS/ha.

Die Formulierungen d.h. die den Wirkstoff der Formel I und gegebenenfalls einen festen oder flüssigen Zusatzstoff enthaltenden Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder Ethylether, Ketone wie Cyclohexanon, stark polare Lösungsmittel wie N-Methyl-2-pyrrolidon, Dimethylsulfoxid oder Dimethylformamid, sowie gegebenenfalls epoxydierte Pflanzenöle wie epoxydiertes Kokosnussöl oder Sojaöl; oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüber-

hinaus kann eine Vielzahl von vorgranulierten Materialien anorganischer oder organischer Natur wie insbesondere Dolomit oder zerkleinerte Pflanzenrückstände verwendet werden. Besonders vorteilhafte, applikationsfördernde Zuschlagstoffe, die zu einer starken Reduktion der Aufwandmenge führen können, sind ferner natürliche (tierische oder pflanzliche) oder synthetische Phospholipide aus der Reihe der Kephaline und Lecithine, wie z.B. Phosphatidylethanolamin, Phosphatidylserin, Phosphatidylcholin, Sphingomyelin, Phosphatidylinosit, Phosphatidylglycerin, Lysolecithin, Plasmalogene oder Cardiolipin, die man beispielsweise aus tierischen oder pflanzlichen Zellen, insbesondere aus Hirn, Herz, Leber, Eidotter oder Sojabohnen gewinnen kann. Verwendbare Handelsmischungen sind z.B. Phosphatidylcholin-Mischungen. Synthetische Phospholipide sind z.B. Dioctanoylphosphatidylcholin und Dipalmitoylphosphatidylcholin.

Als oberflächenaktive Verbindungen kommen je nach Art des zu formulierenden Wirkstoffes der Formel I nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen, als auch wasserlösliche synthetische oberflächenaktive Verbindungen sein.

Als Seifen seien die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können. Ferner sind auch die Fettsäure-methyllaurinsalze zu erwähnen.

Häufiger werden jedoch sog. synthetische Tenside verwendet, insbesondere Fettsulfonate, Fettsulfate, sulfonierte Benzimidazolderivate oder Alkylsulfonate.

Die Fettsulfonate oder -sulfate liegen in der Regel als Alkali-, Erdalkali- oder gegebenenfalls substituierte Ammoniumsalze vor und weisen einen Alkalirest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Ethylenoxyd-Addukten. Die sulfonierten Benzimidazolderivate enthalten vorzugsweise 2-Sulfonsäuregruppen und einen Fettsäurerest mit 8-22 C-Atomen. Alkylarylsulfonate sind z.B. die Na-, Ca- oder Triethanolaminsalze der Dodecylbenzolsulfonsäure, der Dibutylnaphthalinsulfonsäure, oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxyd-Adduktes in Frage.

Als nichtionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 30 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Aethylenglykoläthergruppen und 10 bis 100 g Propylenglykolethergruppen enthaltenden Polyethylenoxidaddukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxydaddukte, Tributylphenoxypolyethylenethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tensiden handelt es sich vor allem um quartäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)ethylammoniumbromid.

Die in der Formulierungstechnik gebräuchlichen Tenside sind u.a. in folgenden Publikationen beschrieben:

"Mc Cutcheon's Detergents and Emulsifiers Annual" BC Publishing Corp., Ringwood New Jersey, 1981;
Helmut Stache "Tensid-Taschenbuch" Carl Hanser-Verlag München/Wien 1981.

Die agrochemischen Zubereitungen enthalten in der Regel 0,1 bis 99%, insbesondere 0,1 bis 95% Wirkstoff der Formel I, 99,9 bis 1%, insbesondere 99,8 bis 5% eines festen oder flüssigen Zusatzstoffes und 0 bis 25%, insbesondere 0,1 bis 25% eines Tensides.

Während als Handelsware eher konzentrierte Mittel bevorzugt werden, verwendet der Endverbaucher in der Regel verdünnte Mittel.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Derartige agrochemische Mittel sind ein Bestandteil der vorliegenden Erfindung.

Die nachfolgenden Beispiele dienen zur näheren Erläuterung der Erfindung, ohne dieselbe einzuschränken. Temperaturen sind in Celsiusgraden angegeben. Prozente und Teile beziehen sich auf das Gewicht. Darüberhinaus werden folgende Symbole verwendet:

h = Stunde; RT = Raumtemperatur; DMSO = Dimethylsulfoxid.

Herstellungsbeispiel

Beispiel H1: Herstellung von

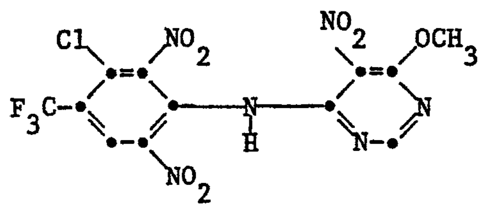

(6.1)

N-(3'-Chlor-2',6'-dinitro-4'-trifluormethylphenyl)-4-amino-(5-nitro-6-methoxy)-pyrimidin

Eine Lösung von 6,1 Teilen 2,4-Dichlor-3,5-dinitrobenzotrifluorid in 50 ml DMSO wird bei Raumtemperatur unter Rühren portionsweise mit 3,2 Teilen 4-Amino-5-nitro-6-methoxypyrimidin versetzt. Zu der Suspension wird unter Kühlen bei Raumtemperatur innerhalb 1/2 h eine Lösung von 4,5 Teilen Kalium-tert.-butylat in 25 ml DMSO getropft. Nach 12 h Rühren bei Raumtemperatur wird das rot gefärbte Reaktionsgemisch auf 700 ml Eiswasser gegossen, mit 3 ml konzentrierter Salzsäure angesäuert und dreimal mit je 200 ml Chloroform extrahiert. Die vereinigten Extrakte werden zweimal mit je 100 ml Wasser gewaschen, über Natriumsulfat getrocknet, filtriert und eingedampft. Die zurückbleibende braune Kristallmasse wird säulenchromatographisch (Kieselgel/

Petrolether: Ethylacetat = 3:2) gereinigt. Nach dem Verdampfen der Laufmittel wird aus Toluol-Cyclohexan umkristallisiert; Smp. 184-186°.

Analog zu den beschriebenen Arbeitsweisen werden auch die nachfolgend aufgeführten Verbindungen der Formel I hergestellt:

Tabelle 1: Verbindungen der Formel

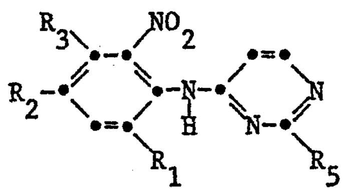

| Verb. Nr. | $R_1$ | $R_2$ | $R_3$ | $R_5$ | Physik. Konstante [°C] |
|---|---|---|---|---|---|
| 1.1 | $NO_2$ | $CF_3$ | Cl | H | Smp.156 |
| 1.2 | $NO_2$ | $CF_3$ | H | $C_3H_7$-n | |
| 1.3 | $NO_2$ | $CF_3$ | Cl | $CH_3$ | |
| 1.4 | $CF_3$ | $NO_2$ | H | $C_6H_5$ | |
| 1.5 | $NO_2$ | $CF_3$ | Cl | $C_3H_7$-n | |
| 1.6 | $NO_2$ | $CF_3$ | Cl | Cl | zähe Masse |
| 1.7 | $CF_3$ | $NO_2$ | H | Cl | zähe Masse |
| 1.8 | $CF_3$ | $NO_2$ | H | $OC_2H_5$ | |
| 1.9 | $NO_2$ | $CF_3$ | Cl | $OC_2H_5$ | |
| 1.10 | $CF_3$ | $NO_2$ | H | $SCH_3$ | |
| 1.11 | $NO_2$ | $CF_3$ | Cl | $OCH_3$ | |

Tabelle 1 (Fortsetzung)

| Verb. Nr. | $R_1$ | $R_2$ | $R_3$ | $R_5$ | Physik. Konstante [°C] |
|---|---|---|---|---|---|
| 1.12 | $NO_2$ | $CF_3$ | Cl | $SCH_3$ | |
| 1.13 | $NO_2$ | $CF_3$ | H | Cl | |
| 1.14 | $NO_2$ | $CF_3$ | Cl | $C_2H_5$ | |
| 1.15 | $NO_2$ | $CF_3$ | Cl | $CF_3$ | Harz |
| 1.16 | $CF_3$ | $NO_2$ | H | $CF_3$ | Harz |
| 1.17 | $NO_2$ | $CF_3$ | Cl | $OC_3H_7$-i | |
| 1.18 | $NO_2$ | $CF_3$ | H | $OC_2H_5$ | |
| 1.19 | $NO_2$ | $CF_3$ | Cl | $CH_2OCH_3$ | |
| 1.20 | $NO_2$ | $CF_3$ | Cl | $SCH_2-C_6H_5$ | |
| 1.21 | $NO_2$ | $CF_3$ | H | $CF_3$ | Harz |
| 1.22 | $NO_2$ | $CF_3$ | Cl | $-N(CH_3)_2$ | |

Tabelle 2: Verbindungen der Formel

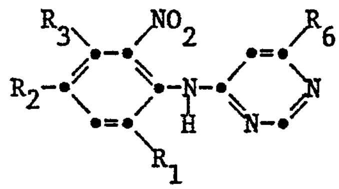

| Verb. Nr. | $R_1$ | $R_2$ | $R_3$ | $R_6$ | Physik. Kostante [°C] |
|---|---|---|---|---|---|
| 2.1 | $NO_2$ | $CF_3$ | H | H | |
| 2.2 | $NO_2$ | $CF_3$ | Cl | Cl | Smp. 97-99 |
| 2.3 | $CF_3$ | $NO_2$ | H | $OC_6H_5$ | |
| 2.4 | $NO_2$ | $CF_3$ | Cl | $OCH_3$ | Smp. 128-129 |
| 2.5 | $NO_2$ | $CF_3$ | Cl | $SCH_3$ | Smp. 135-137 |
| 2.6 | $NO_2$ | $CF_3$ | Cl | $OCH_2CF_3$ | Smp. 98-100 |
| 2.7 | $CF_3$ | $NO_2$ | H | $CF_3$ | |
| 2.8 | $NO_2$ | $CF_3$ | Cl | $-C(CH_3)_3$ | |
| 2.9 | $CF_3$ | $NO_2$ | H | Cl | Harz |

Tabelle 2 (Fortsetzung)

| Verb. Nr. | $R_1$ | $R_2$ | $R_3$ | $R_6$ | Physik. Konstante [°C] |
|---|---|---|---|---|---|
| 2.10 | $NO_2$ | $CF_3$ | Cl | $-N(C_2H_5)_2$ | |
| 2.11 | $NO_2$ | $CF_3$ | Cl | $C_2H_5$ | |
| 2.12 | $NO_2$ | $CF_3$ | Cl | $-N(CH_3)_2$ | |
| 2.13 | $NO_2$ | $CF_3$ | Cl | $C_6H_5$ | |
| 2.14 | $NO_2$ | $CF_3$ | Cl | 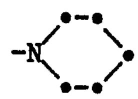 | |
| 2.15 | $NO_2$ | $CF_3$ | H | $-N(C_3H_7\text{-n})_2$ | |
| 2.16 | $NO_2$ | $CF_3$ | Cl | $CF_3$ | zähe Masse |
| 2.17 | $NO_2$ | $CF_3$ | Cl | $CH_3$ | |
| 2.18 | $NO_2$ | $CF_3$ | H | $OC_4H_9\text{-n}$ | |
| 2.19 | $NO_2$ | $CF_3$ | Cl | $OC_3H_7\text{-i}$ | |
| 2.20 | $NO_2$ | $CF_3$ | Cl | $OCH_3$ | |
| 2.21 | $CF_3$ | $NO_2$ | H | $OC_2H_5$ | |

Tabelle 3: Verbindungen der Formel

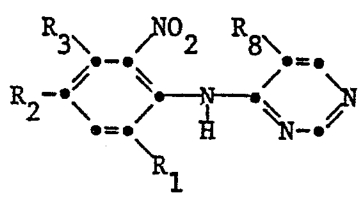

| Verb. Nr. | $R_1$ | $R_2$ | $R_3$ | $R_8$ | Physik. Konstante [°C] |
|---|---|---|---|---|---|
| 3.1 | $CF_3$ | $NO_2$ | H | H | semikristallin |
| 3.2 | $NO_2$ | $CF_3$ | Cl | CN | |
| 3.3 | $NO_2$ | $CF_3$ | H | Cl | Harz |
| 3.4 | $NO_2$ | $CF_3$ | Cl | Br | |
| 3.5 | $CF_3$ | $NO_2$ | H | CN | |
| 3.6 | $NO_2$ | $CF_3$ | Cl | Cl | |

Tabelle 3 (Fortsetzung)

| Verb. Nr. | $R_1$ | $R_2$ | $R_3$ | $R_8$ | Physik. Konstante [°C] |
|---|---|---|---|---|---|
| 3.7 | $NO_2$ | $CF_3$ | Cl | Cl | Harz |
| 3.8 | $CF_3$ | $NO_2$ | H | Br | |
| 3.9 | $NO_2$ | $CF_3$ | Cl | $C_3H_7$-i | |
| 3.10 | $NO_2$ | $CF_3$ | Cl | $CH_2OC_2H_5$ | |
| 3.11 | $NO_2$ | $CF_3$ | Cl | $CH_2OC_6H_5$ | |
| 3.12 | $NO_2$ | $CF_3$ | Cl | $C_2H_5$ | |
| 3.13 | $CF_3$ | $NO_2$ | H | $CH_3$ | |
| 3.14 | $NO_2$ | $CF_3$ | Cl | $OCH_3$ | |
| 3.15 | $NO_2$ | $CF_3$ | Cl | $CH_3$ | |
| 3.16 | $CF_3$ | $NO_2$ | H | $CH_2OCH_3$ | |
| 3.17 | $NO_2$ | $CF_3$ | Cl | $C_6H_5$ | |
| 3.18 | $NO_2$ | $CF_3$ | Cl | $CH_2C_6H_4Cl(4)$ | |
| 3.19 | $NO_2$ | $CF_3$ | Cl | $C_6H_{13}$-n | |
| 3.20 | $NO_2$ | $CF_3$ | Cl | $C_5H_4Cl(4)$ | |
| 3.21 | $NO_2$ | $CF_3$ | Cl | $CH_2Cl$ | |
| 3.22 | $NO_2$ | $CF_3$ | Cl | $OC_6H_3Cl_2(3,4)$ | |

Tabelle 4: Verbindungen der Formel

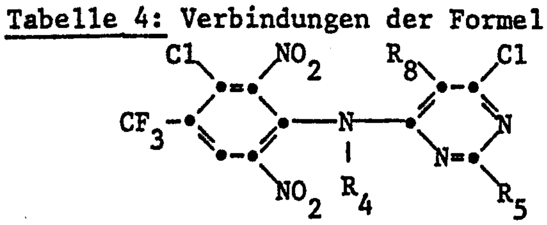

| Verb. Nr. | $R_4$ | $R_8$ | $R_5$ |
|---|---|---|---|
| 4.1 | $C(O)CH{=}CH_2$ | F | $SCH_3$ |
| 4.2 | $C(O)CH{=}CH_2$ | Cl | CN |
| 4.3 | $C(O)CCl{=}CCl_2$ | F | $SCH_3$ |
| 4.4 | C(O)Cyclopropyl | F | $SCH_3$ |
| 4.5 | $C(O)CH{=}CH_2$ | CN | $CF_3$ |
| 4.6 | $C(O)C_6H_4Cl(4)$ | F | $SCH_3$ |
| 4.7 | 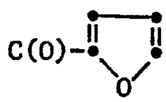 | $OCH_3$ | CN |
| 4.8 | 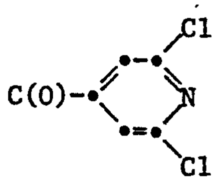 | F | $SCH_3$ |

Tabelle 5: Verbindungen der Formel

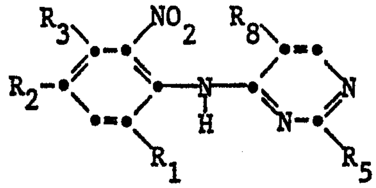

| Verb. Nr. | $R_1$ | $R_2$ | $R_3$ | $R_5$ | $R_8$ | Physik. Konst. [°C] |
|---|---|---|---|---|---|---|
| 5.1 | $NO_2$ | $CF_3$ | Cl | $OCH_3$ | Cl | Smp. 157-158 |
| 5.2 | $NO_2$ | $CF_3$ | Cl | $CH_3$ | $C_4H_9$-n | |
| 5.3 | $NO_2$ | $CF_3$ | Cl | Cl | Cl | Smp. 176-177 |
| 5.4 | $NO_2$ | $CF_3$ | Cl | Cl | F | Smp. 167-168 |
| 5.5 | $NO_2$ | $CF_3$ | Cl | $CH_3$ | $CH_2N(CH_3)_2$ | |
| 5.6 | $NO_2$ | $CF_3$ | Cl | $OCH_3$ | F | Smp. 158-159 |
| 5.7 | $NO_2$ | $CF_3$ | Cl | $SCH_3$ | F | |
| 5.8 | $CF_3$ | $NO_2$ | H | $CH_3$ | $C_6H_{13}$-n | |
| 5.9 | $NO_2$ | $CF_3$ | Cl | $C_3H_7$ | $CH_2Br$ | |
| 5.10 | $NO_2$ | $CF_3$ | Cl | $CH_2C_6H_5$ | $CH_2Br$ | |
| 5.11 | $NO_2$ | $CF_3$ | Cl | $CH_3$ | $CH_2SCN$ | |
| 5.12 | $CF_3$ | $NO_2$ | H | $-N(CH_3)_2$ | CN | |
| 5.13 | $NO_2$ | $CF_3$ | Cl | Cl | $CH_2Cl$ | |
| 5.14 | $CF_3$ | $NO_2$ | H | $CH_3$ | $CH_2CN$ | |
| 5.15 | $NO_2$ | $CF_3$ | Cl | $C_2F_5$ | CN | |
| 5.16 | $NO_2$ | $CF_3$ | Cl | $SO_2C_2H_5$ | $CH_3$ | |
| 5.17 | $CF_3$ | $NO_2$ | H | $SCH_3$ | $CH_3$ | |
| 5.18 | $NO_2$ | $CF_3$ | Cl | $CF_3$ | CN | Harz |
| 5.19 | $NO_2$ | $CF_3$ | Cl | $OCH_3$ | CN | |
| 5.20 | $NO_2$ | $CF_3$ | Cl | $SC_2H_5$ | $C_6H_5$ | |
| 5.21 | $CF_3$ | $NO_2$ | H | $CF_3$ | CN | |
| 5.22 | $NO_2$ | $CF_3$ | Cl | Cl | $OCH_3$ | |
| 5.23 | $NO_2$ | $CF_3$ | Cl | $CF_3$ | $CH_2OC_2H_5$ | |
| 5.24 | $CF_3$ | $NO_2$ | H | $C_3F_7$-n | CN | |
| 5.25 | $NO_2$ | $CF_3$ | Cl | $CH_2OCH_3$ | $CH_2OC_2H_5$ | |
| 5.26 | $NO_2$ | $CF_3$ | Cl | $SO_2CH_3$ | CN | |

Tabelle 5 (Fortsetzung)

| Verb. Nr. | $R_1$ | $R_2$ | $R_3$ | $R_5$ | $R_8$ | Physik. Konst. [°C] |
|---|---|---|---|---|---|---|
| 5.27 | $NO_2$ | $CF_3$ | Cl | 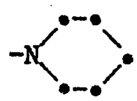 | $NO_2$ | |
| 5.28 | $NO_2$ | $CF_3$ | Cl | $C_2F_5$ | Br | |
| 5.29 | $NO_2$ | $CF_3$ | Cl | $CF_3$ | $CH_2Br$ | |
| 5.30 | $NO_2$ | $CF_3$ | Cl | $SCH_3$ | $CH_2Cl$ | |
| 5.31 | $NO_2$ | $CF_3$ | Cl | $SC_2H_5$ | CN | |
| 5.32 | $CF_3$ | $NO_2$ | H | $CF_3$ | $CH_2Cl$ | |
| 5.33 | $NO_2$ | $CF_3$ | Cl | $SC_2H_5$ | F | |
| 5.34 | $NO_2$ | $CF_3$ | Cl | $C_5H_9$-n | CN | |
| 5.35 | $NO_2$ | $CF_3$ | Cl | $-N(CH_3)_2$ | Br | |
| 5.36 | $NO_2$ | $CF_3$ | Cl | $SCH_3$ | $NO_2$ | |
| 5.37 | $CF_3$ | $NO_2$ | H | $C_4H_9$-n | CN | |
| 5.38 | $CF_3$ | $NO_2$ | H | Cl | $NO_2$ | |
| 5.39 | $NO_2$ | $CF_3$ | H | $C_6H_4Cl(4)$ | CN | |
| 5.40 | $CF_3$ | $NO_2$ | H | $SCH_2C_6H_5$ | CN | |
| 5.41 | $NO_2$ | $CF_3$ | Cl | $-CH=CH_2$ | CN | |
| 5.42 | $NO_2$ | $CF_3$ | Cl | $SCH_3$ | $CH_2Br$ | |
| 5.43 | $CF_3$ | $NO_2$ | H | Cl | $CH_2Br$ | |
| 5.44 | $NO_2$ | $CF_3$ | H | $SCH_2C_6H_5$ | $CH-CH=CH_2$ | |
| 5.45 | $NO_2$ | $CF_3$ | Cl | $CH_2F$ | $CH_3$ | |
| 5.46 | $NO_2$ | $CF_3$ | Cl | $OCH_3$ | $NO_2$ | Smp. 178-179 |
| 5.47 | $NO_2$ | $CF_3$ | Cl | $OC_2H_5$ | Cl | Smp. 149-151 |
| 5.48 | $NO_2$ | $CF_3$ | Cl | $OCH_2CF_3$ | Cl | Smp. 146-147 |
| 5.49 | $NO_2$ | $CF_3$ | Cl | $O(CH_2)_2OCH_3$ | $NO_2$ | Smp. 125-126 |
| 5.50 | $NO_2$ | $CF_3$ | Cl | $OCH_2CF_3$ | $NO_2$ | Smp. 138-140 |
| 5.51 | $NO_2$ | $CF_3$ | Cl | $OCH_2CF_3$ | F | Smp. 135-138 |

Tabelle 5 (Fortsetzung)

| Verb. Nr. | $R_1$ | $R_2$ | $R_3$ | $R_5$ | $R_8$ | Physik. Konst. [°C] |
|---|---|---|---|---|---|---|
| 5.52 | $NO_2$ | $CF_3$ | Cl | $SCH_3$ | $CH_3$ | |
| 5.53 | $NO_2$ | $CF_3$ | H | $SCH_3$ | $CH_3$ | |
| 5.54 | $NO_2$ | $CF_3$ | H | $SCH_3$ | Cl | |
| 5.55 | $NO_2$ | $CF_3$ | Cl | $OCH_3$ | $CH_3$ | |
| 5.56 | $CF_3$ | $NO_2$ | H | $OCH_3$ | $CH_3$ | |
| 5.57 | $CF_3$ | $NO_2$ | H | Cl | $CH_3$ | |
| 5.58 | $NO_2$ | $CF_3$ | H | $OCH_3$ | $CH_3$ | |
| 5.59 | $CF_3$ | $NO_2$ | H | $OCH_2-CH=CH_2$ | Cl | |
| 5.60 | $NO_2$ | $CF_3$ | Cl | Cl | $CH_3$ | |
| 5.61 | $NO_2$ | $CF_3$ | H | Cl | $CH_3$ | |
| 5.62 | $CF_3$ | $NO_2$ | H | $SCH_3$ | Cl | |
| 5.63 | $NO_2$ | $CF_3$ | H | $OCH_2CH=CH_2$ | Cl | |
| 5.64 | $NO_2$ | $CF_3$ | Cl | $SCH_3$ | Cl | |
| 5.65 | $NO_2$ | $CF_3$ | Cl | $OCH_2CH=CH_2$ | Cl | |

Tabelle 6: Verbindungen der Formel

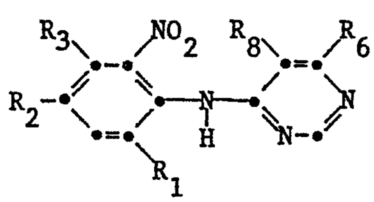

| Verb. Nr. | $R_1$ | $R_2$ | $R_3$ | $R_6$ | $R_8$ | Physik. Konst. [°C] |
|---|---|---|---|---|---|---|
| 6.1 | $NO_2$ | $CF_3$ | Cl | $OCH_3$ | $NO_2$ | Smp. 184-186 |
| 6.2 | $NO_2$ | $CF_3$ | Cl | $-NH_2$ | $NO_2$ | Smp. 184-189 |
| 6.3 | $CF_3$ | $NO_2$ | H | $CH_2OC_2H_5$ | $CH_3$ | |
| 6.4 | $NO_2$ | $CF_3$ | Cl | Cl | $NO_2$ | |
| 6.5 | $NO_2$ | $CF_3$ | Cl | $CH_3$ | CN | |
| 6.6 | $NO_2$ | $CF_3$ | Cl | $-N(C_3H_7-n)_2$ | $NO_2$ | |
| 6.7 | $CF_3$ | $NO_2$ | H | $OC_2H_4OC_2H_5$ | $C_6H_5$ | |

Tabelle 6 (Fortsetzung)

| Verb. Nr. | $R_1$ | $R_2$ | $R_3$ | $R_6$ | $R_8$ | Physik. Konst. [°C] |
|---|---|---|---|---|---|---|
| 6.8 | $NO_2$ | $CF_3$ | Cl | Cl | $OC_4H_9$-n | |
| 6.9 | $NO_2$ | $CF_3$ | Cl | F | $NO_2$ | |
| 6.10 | $NO_2$ | $CF_3$ | Cl | Cl | $CH_3$ | |
| 6.11 | $NO_2$ | $CF_3$ | Cl | Cl | $OCH_3$ | |
| 6.12 | $CF_3$ | $NO_2$ | H | $OC_2H_5$ | $NO_2$ | |
| 6.13 | $NO_2$ | $CF_3$ | Cl | Cl | $OC_2H_5$ | |
| 6.14 | $NO_2$ | $CF_3$ | Cl | $CH_3$ | Br | |
| 6.15 | $CF_3$ | $NO_2$ | H | $CH_3$ | Cl | |
| 6.16 | $NO_2$ | $CF_3$ | Cl | Cl | $C_6H_5$ | |
| 6.17 | $NO_2$ | $CF_3$ | Cl | $C_2H_4OC_2H_5$ | $OC_4H_9$-n | |
| 6.18 | $NO_2$ | $CF_3$ | Cl | Cl | $OC_3H_7$-i | |
| 6.19 | $CF_3$ | $NO_2$ | H | $CH_3$ | CN | |
| 6.20 | $CF_3$ | $NO_2$ | H | Cl | $OC_3H_7$-i | |
| 6.21 | $CF_3$ | $NO_2$ | H | $CH_3$ | J | |
| 6.22 | $NO_2$ | $CF_3$ | H | Cl | $OC_2H_5$ | |
| 6.23 | $NO_2$ | $CF_3$ | Cl | $SCH_3$ | $NO_2$ | Smp. 185 |
| 6.24 | $NO_2$ | $CF_3$ | Cl | $OCH_2CF_3$ | $NO_2$ | Smp. 164-167 |
| 6.25 | $NO_2$ | $CF_3$ | Cl | $OCH_3$ | Cl | |
| 6.26 | $CF_3$ | $NO_2$ | H | $OCH_2CF_3$ | Cl | |
| 6.27 | $NO_2$ | $CF_3$ | Cl | $OCH_2$-CH=$CH_2$ | Cl | |
| 6.28 | $NO_2$ | $CF_3$ | Cl | $SCH_3$ | Cl | |
| 6.29 | $NO_2$ | $CF_3$ | H | Cl | Cl | |

Tabelle 7: Verbindungen der Formel

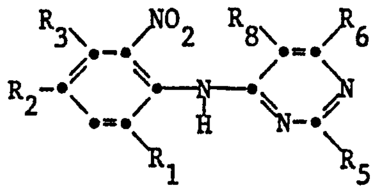

| Verb. Nr. | $R_1$ | $R_2$ | $R_3$ | $R_5$ | $R_6$ | $R_8$ | Physik. Konst. [°C] |
|---|---|---|---|---|---|---|---|
| 7.1 | $NO_2$ | $CF_3$ | Cl | $SCH_3$ | $OCH_3$ | $NO_2$ | Smp. 181-183 |
| 7.2 | $NO_2$ | $CF_3$ | Cl | Cl | Cl | $NO_2$ | |
| 7.3 | $NO_2$ | $CF_3$ | Cl | $CH_3$ | Cl | $C_2H_5$ | Smp. 134-136 |
| 7.4 | $NO_2$ | $CF_3$ | Cl | Cl | $CH_3$ | $NO_2$ | |
| 7.5 | $NO_2$ | $CF_3$ | Cl | $CH_2CN$ | $CH_2CN$ | CN | |
| 7.6 | $NO_2$ | $CF_3$ | Cl | F | F | F | |
| 7.7 | $CF_3$ | $NO_2$ | H | Cl | Cl | $NO_2$ | |
| 7.8 | $CF_3$ | $NO_2$ | H | Cl | Cl | Br | |
| 7.9 | $NO_2$ | $CF_3$ | Cl | $CH_3$ | $CH_3$ | $SO_2CH_3$ | |
| 7.10 | $NO_2$ | $CF_3$ | Cl | Cl | Cl | Br | |
| 7.11 | $NO_2$ | $CF_3$ | Cl | $SCH_3$ | Cl | Br | |
| 7.12 | $NO_2$ | $CF_3$ | Cl | Cl | Cl | $CH_3$ | Smp. 184-187 |
| 7.13 | $CF_3$ | $NO_2$ | H | $SCH_3$ | Cl | $C_8H_{17}$-n | |
| 7.14 | $CF_3$ | $NO_2$ | H | Br | Br | $CH_3$ | |
| 7.15 | $NO_2$ | $CF_3$ | Cl | Cl | Cl | $CH_2C_6H_5$ | |
| 7.16 | $NO_2$ | $CF_3$ | Cl | Cl | $CH_3$ | $CH_3$ | |
| 7.17 | $CF_3$ | $NO_2$ | H | $CF_3$ | $CF_3$ | CN | |
| 7.18 | $NO_2$ | $CF_3$ | Cl | Cl | Cl | Cl | |
| 7.19 | $CF_3$ | $NO_2$ | H | Cl | Cl | $CH_3$ | Smp. 207-208 |
| 7.20 | $NO_2$ | $CF_3$ | Cl | Br | Br | $CH_3$ | |
| 7.21 | $NO_2$ | $CF_3$ | Cl | $CH_3$ | Cl | $CH_2N(CH_3)_2$ | |
| 7.22 | $CF_3$ | $NO_2$ | H | Cl | Cl | Cl | |
| 7.23 | $CF_3$ | $NO_2$ | H | Cl | Cl | $C_2H_5$ | |
| 7.24 | $CF_3$ | $NO_2$ | H | $SCH_3$ | $CH_3$ | $NO_2$ | |
| 7.25 | $NO_2$ | $CF_3$ | H | Cl | Cl | Cl | |
| 7.26 | $NO_2$ | $CF_3$ | Cl | $-N(CH_3)_2$ | Cl | CN | |
| 7.27 | $NO_2$ | $CF_3$ | Cl | $CF_3$ | $CF_3$ | CN | |

Tabelle 7 (Fortsetzung)

| Verb. Nr. | $R_1$ | $R_2$ | $R_3$ | $R_5$ | $R_6$ | $R_8$ | Physik. Konst. [°C] |
|---|---|---|---|---|---|---|---|
| 7.28 | $CF_3$ | $NO_2$ | H | Cl | $CH_3$ | $C_2H_5$ | |
| 7.29 | $NO_2$ | $CF_3$ | Cl | $SCH_3$ | $OCH_3$ | Br | |
| 7.30 | $NO_2$ | $CF_3$ | Cl | $SCH_3$ | Cl | $C_4H_9$-n | |
| 7.31 | $NO_2$ | $CF_3$ | Cl | Cl | Cl | $C_2H_5$ | |
| 7.32 | $NO_2$ | $CF_3$ | Cl | $SCH_3$ | $CH_3$ | CN | |
| 7.33 | $NO_2$ | $CF_3$ | Cl | $SCH_3$ | Cl | $CH_3$ | Smp. 169-172 |
| 7.34 | $NO_2$ | $CF_3$ | Cl | $CH_2OCH_3$ | Cl | $C_2H_5$ | |
| 7.35 | $NO_2$ | $CF_3$ | Cl | $SCH_3$ | $NH_2$ | $NO_2$ | Smp. 250 (Zers.) |
| 7.36 | $NO_2$ | $CF_3$ | Cl | $OCH_3$ | $OCH_3$ | $NO_2$ | Smp. 164-166 |
| 7.37 | $NO_2$ | $CF_3$ | H | $SCH_3$ | Cl | $CH_3$ | Smp. 213-216 |
| 7.38 | $CF_3$ | $NO_2$ | H | $SCH_3$ | Cl | $CH_3$ | Smp. 150-160 |
| 7.39 | $NO_2$ | $CF_3$ | Cl | $CH_3$ | $CH_3$ | J | |
| 7.40 | $NO_2$ | $CF_3$ | Br | $CH_3$ | $CH_3$ | J | |
| 7.41 | $CF_3$ | $NO_2$ | H | $CH_3$ | $CH_3$ | J | |
| 7.42 | $NO_2$ | $CF_3$ | Cl | $SCH_3$ | Cl | Cl | |
| 7.43 | $CF_3$ | $NO_2$ | H | $C_2H_5$ | $OCH_3$ | Cl | |
| 7.44 | $NO_2$ | $CF_3$ | Cl | $OCH_3$ | Cl | Cl | |
| 7.45 | $NO_2$ | $CF_3$ | Cl | $C_2H_5$ | $SCH_3$ | Cl | |
| 7.46 | $NO_2$ | $CF_3$ | Cl | $OCH_2CF_3$ | Cl | Cl | |
| 7.47 | $NO_2$ | $CF_3$ | H | $OCH_2-CH=CH_2$ | Cl | Cl | |
| 7.48 | $NO_2$ | $CF_3$ | Cl | $C_2H_5$ | Cl | Cl | |
| 7.49 | $NO_2$ | $CF_3$ | Cl | $OCH_2-CH\equiv CH$ | Cl | Cl | |
| 7.50 | $NO_2$ | $CF_3$ | Cl | Cl | $CF_3$ | Cl | |
| 7.51 | $NO_2$ | $CF_3$ | Cl | $OCH_3$ | $CF_3$ | Cl | |
| 7.52 | $NO_2$ | $CF_3$ | Cl | $SCH_3$ | $CF_3$ | Cl | |

Formulierungsbeispiele für flüssige Wirkstoffe der Formel I

(% = Gewichtsprozent

| F1. Emulsions-Konzentrate | a) | b) | c) |
| --- | --- | --- | --- |
| Wirkstoff aus den Tabellen | 25% | 40% | 50% |
| Ca-Dodecylbenzolsulfonat | 5% | 8% | 6% |
| Ricinusöl-polyethylenglykolether (36 Mol Ethylenoxid) | 5% | - | - |
| Tributylphenoyl-polyethylenglykol-ether (30 Mol Ethylenoxid) | - | 12% | 4% |
| Cyclohexanon | - | 15% | 20% |
| Xylolgemisch | 65% | 25% | 20% |

Aus solchen Konzentraten können durch Verdünnen mit Wasser Emulsionen jeder gewünschten Konzentration hergestellt werden.

| F2. Lösungen | a) | b) | c) | d) |
| --- | --- | --- | --- | --- |
| Wirkstoff aus den Tabellen | 80% | 10% | 5% | 95% |
| Ethylenglykol-monomethyl-ether | 20% | - | - | - |
| Polyethylenglykol M G 400 | - | 70% | - | - |
| N-Methyl-2-pyrrolidon | - | 20% | - | - |
| Epoxydiertes Kokosnussöl | - | - | 1% | 5% |
| Benzin (Siedegrenzen 160-190°C) | - | - | 94% | - |

(MG = Molekulargewicht)

Die Lösungen sind zur Anwendung in Form kleinster Tropfen geeignet.

| F3. Granulate | a) | b) |
| --- | --- | --- |
| Wirkstoff aus den Tabellen | 5% | 10% |
| Kaolin | 94% | - |
| Hochdisperse Kieselsäure | 1% | - |
| Attapulgit | - | 90% |

Der Wirkstoff wird in Methylenchlorid gelöst, auf den Träger aufgesprüht und das Lösungsmittel anschliessend im Vakuum abgedampft.

| F4. Stäubemittel | a) | b) |
|---|---|---|
| Wirkstoff aus den Tabellen | 2% | 5% |
| Hochdisperse Kieselsäure | 1% | 5% |
| Talkum | 97% | - |
| Kaolin | - | 90% |

Durch inniges Vermischen auf Trägerstoffe mit dem Wirkstoff erhält man gebauchsfertige Stäubemittel.

Formulierungsbeispiele für feste Wirkstoffe der Formel I (% = Gewichtsprozent)

| F5. Spritzpulver | a) | b) | c) |
|---|---|---|---|
| Wirkstoff aus den Tabellen | 25% | 50% | 75% |
| Na-Ligninsulfonat | 5% | 5% | - |
| Na-Laurylsulfat | 3% | - | 5% |
| Na-Diisobutylnaphthalinsulfonat | - | 6% | 10% |
| Octylphenolpolyethylenglykolether (7-8 Mol Ethylenoxid) | - | 2% | - |
| Hochdisperse Kieselsäure | 5% | 10% | 10% |
| Kaolin | 62% | 27% | - |

Der Wirkstoff wird mit den Zusatzstoffen gut vermischt und in einer geeigneten Mühle gut vermahlen. Man erhält Spritzpulver, die sich mit Wasser zu Suspensionen jeder gewünschten Konzentration verdünnen lassen.

F6. Emulsions-Konzentrat

| | |
|---|---|
| Wirkstoff aus den Tabellen | 10% |
| Octylphenolpolyethylenglykolether (4-5 Mol Ethylenoxid) | 3% |
| Ca-Dodecylbenzolsulfonat | 3% |
| Ricinusölpolyglykolether (35 Mol Ethylenoxid) | 4% |
| Cyclohexanon | 30% |
| Xylolgemisch | 50% |

Aus diesem Konzentrat können durch Verdünnen mit Wasser Emulsionen jeder gewünschten Konzentration hergestellt werden.

| F7. Stäubemittel | a) | b) |
|---|---|---|
| Wirkstoff aus den Tabellen | 5% | 8% |
| Talkum | 95% | - |
| Kaolin | - | 92% |

Man erhält anwendungsfertige Stäubemittel, indem der Wirkstoff mit den Träger vermischt auf einer geeigneten Mühle vermahlen wird.

F8. Extruder Granulat

| | |
|---|---|
| Wirkstoff aus den Tabellen | 10% |
| N-Ligninsulfonat | 2% |
| Carboxymethylcellulose | 1% |
| Kaolin | 87% |

Der Wirkstoff wird mit den Zusatzstoffen vermischt, vermahlen und mit Wasser angefeuchtet. Dieses Gemisch wird extrudiert und anschliessend im Luftstrum getrocknet.

F9. Umhüllungs-Granulat

| | |
|---|---|
| Wirkstoff aus den Tabellen | 3% |
| Polyethylenglykol (M G 200) | 3% |
| Kaolin | 94% |

(MG = Molekulargewicht)

Der fein gemahlene Wirkstoff wird in einem Mischer auf das mit Polyethylenglykol angefeuchtete Kaolin gleichmässig aufgetragen. Auf diese Weise erhält man staubfreie Umhüllungs-Granulate.

F10. Suspensions-Konzentrat

| | |
|---|---|
| Wirkstoff aus den Tabellen | 40% |
| Ethylenglykol | 10% |
| Nonylphenolpolyethylenglykolether (15 Mol Ethylenoxid) | 6% |
| N-Ligninsulfonat | 10% |
| Carboxymethylcellulose | 1% |
| 37%ige wässrige Formaldehyd-Lösung | 0,2% |
| Silikonöl in Form einer 75%igen wässrigen Emulsion | 0,8% |
| Wasser | 32% |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Biologische Beispiele:

Beispiel B1: Wirkung gegen Puccinia graminis auf Weizen

a) Residual-protektive Wirkung

Weizenpflanzen wurden 6 Tage nach der Aussaat mit einer aus Spritzpulver des Wirkstoffes hergestellten Spritzbrühe (0,02% Aktivsubstanz) besprüht. Nach 24 Stunden wurden die behandelten Pflanzen mit einer

Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

b) Systemische Wirkung

Zu Weizenpflanzen wurden 5 Tage nach Aussaat eine aus Spritzpulver des Wirkstoffes hergestellte Spritzbrühe gegossen (0,006% Aktivsubstanz bezogen auf das Bodenvolumen). Nach 48 Stunden wurden die behandelten Pflanzen mit einer Uredosporensuspension des Pilzes infiziert. Nach einer Inkubation während 48 Stunden bei 95-100% relativer Luftfeuchtigkeit und ca. 20°C wurden die infizierten Pflanzen in einem Gewächshaus bei ca. 22°C aufgestellt. Die Beurteilung der Rostpustelnentwicklung erfolgte 12 Tage nach der Infektion.

Verbindungen aus den Tabellen zeigten gegen Puccinia-Pilze eine sehr gute Wirkung. Unbehandelte aber infizierte Kontrollpflanzen zeigten einen Puccinia-Befall von 100%. Unter anderen hemmten Verbindungen aus den Tabellen 1, 2, 5 und 7 den Puccinia-Befall auf 0 bis 5 %.

Beispiel B2: Wirkung gegen Cersopora arachidicola auf Erdnusspflanzen

Residual-protektive Wirkung

10-15 cm hohe Erdnusspflanzen wurden mit einer aus Spritzpulver der Wirksubstanz hergestellten Spritzbrühe (0,006 Aktivsubstanz) besprüht und 48 Stunden später mit einer Konidiensuspension des Pilzes infiziert. Die infizierten Pflanzen wurden während 72 Stunden bei ca. 21°C und hoher Luftfeuchtigkeit inkubiert und anschliessend bis zum Auftreten der typischen Blattflecken in einem Gewächshaus aufgestellt.

 

Die Beurteilung der fungiziden Wirkung erfolgt 12 Tage nach der Infektion basierend auf Anzahl und Grösse der auftretenden Flecken.

Im Vergleich zu unbehandelten, aber infizierten Kontrollpflanzen (Anzahl und Grösse der Flecken = 100%), zeigten Erdnusspflanzen, die mit Wirkstoffen aus den Tabellen behandelt wurden, einen stark reduzierten Cercospora-Befall. So verhinderten die Verbindungen 1.1, 1.6, 1.7, 1.16, 1.21, 2.2, 2.5, 2.6, 2.9, 2.16, 3.1, 3.3, 3.7, 5.1, 5.3, 5.6, 5.18, 5.46, 5.48, 5.50, 5.51, 6.1, 6.2, 6.23, 6.24, 7.1, 7.3, 7.12, 7.36, 7.37 und 7.38 in obigen Versuchen das Auftreten von Flecken fast vollständig (0-10%).

Beispiel B3: Wirkung gegen Erysiphae graminis auf Gerste

a) Residual-protektive Wirkung

Ca. 8 cm hohe Gerstenpflanzen wurden mit einer aus Spritzpulver des Wirkstoffes hergestellten Spritzbrühe (0,002% Aktivsubstanz) besprüht. Nach 3-4 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

b) Systemische Wirkung

Zu ca. 8 cm hohen Gerstenpflanzen wurde eine aus Spritzpulver des Wirkstoffes hergestellte Spritzbrühe gegossen (0,006% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit Konidien des Pilzes bestäubt. Die infizierten Gerstenpflanzen wurden in einem Gewächshaus bei ca. 22°C aufgestellt und der Pilzbefall nach 10 Tagen beurteilt.

Verbindungen der Formel I zeigten gute Wirkung gegen Erysiphe-Pilze. Unbehandelte, aber infizierte Kontrollpflanzen zeigten einen Erysiphe-Befall von 100%. Unter anderen Verbindungen aus den

Tabellen hemmten die Verbindungen Nr. 1.1, 1.6, 1.7, 1.16, 1.21, 2.2, 2.5, 2.6, 2.9, 2.16, 3.1, 3.3, 3.7, 5.1, 5.3, 5.6, 5.18, 5.46, 5.48, 5.50, 5.51, 6.1, 6.2, 6.23, 6.24, 7.1, 7.3, 7.12, 7.36, 7.37 und 7.38 den Pilzbefall bei Gerste auf weniger als 30%.

Beispiel B4: Residual-protektive Wirkung gegen Venturia inaequalis auf Apfeltrieben.

Apfelstecklinge mit 10-20 cm langen Frischtrieben wurden mit einer aus Spritzpulver des Wirkstoffes hergestellten Spritzbrühe (0,006% Aktivsubstanz) besprüht. Nach 24 Stunden wurden die behandelten Pflanzen mit einer Konidiensuspension des Pilzes infiziert. Die Pflanzen wurden dann während 5 Tagen bei 90-100% relativer Luftfeuchtigkeit inkubiert und während 10 weiteren Tagen in einem Gewächshaus bei 20-24°C aufgestellt. Der Schorfbefall wurde 15 Tage nach der Infektion beurteilt. Verbindungen aus den Tabellen hemmten den Krankheitsbefall auf weniger als 25%. Unbehandelte aber infizierte Kontrolltriebe wurden dagegen 100%ig befallen.

Beispiel B5: Wirkung gegen Botrytis cinerea auf Bohnen

Residual protektive Wirkung

Ca. 10 cm hohe Bohnen-Pflanzen wurden mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach einer Inkubation der infizierten Pflanzen während 3 Tagen bei 95-100% relativer Luftfeuchtigkeit und 21°C erfolgte die Beurteilung des Pilzbefalls. Die Verbindungen aus der Tabelle hemmten in vielen Fällen die Pilzinfektion sehr stark. Bei einer Konzentration von 0,02% erwiesen sich z.B. die Verbindungen 1.1, 1.6, 1.7, 1.15, 2.4, 2.6, 2.16, 3.3, 3.7, 5.3, 5.6, 5.18, 5.46, 5.49, 6.2, 7.1, 7.12 und 7.33 als voll wirksam. Der Krankheitsbefall lag im allgemeinen bei 0 bis 8%. Der Botrytis-Befall unbehandelter aber infizierter Bohnenpflanzen betrug 100%.

Beispiel B6: Wirkung gegen Phytophthora infestans auf Tomatenpflanzen

a) Residual-protektive Wirkung

Tomatenpflanzen wurden nach 3-wöchiger Anzucht mit einer aus Spritzpulver des Wirkstoffes hergestellten Spritzbrühe (0,06% Aktivsubstanz) besprüht. Nach 24 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100% relativer Luftfeuchtigkeit und 20°C.

b) Systemische Wirkung

Zu Tomatenpflanzen wurde nach 3-wöchiger Anzucht eine aus Spritzpulver des Wirkstoffes hergestellte Spritzbrühe gegossen (0,06% Aktivsubstanz bezogen auf das Erdvolumen). Es wurde dabei darauf geachtet, dass die Spritzbrühe nicht mit den oberirdischen Pflanzenteilen in Berührung kam. Nach 48 Stunden wurden die behandelten Pflanzen mit einer Sporangiensuspension des Pilzes infiziert. Die Beurteilung des Pilzbefalls erfolgte nach einer Inkubation der infizierten Pflanzen während 5 Tagen bei 90-100% relativer Luftfeuchtigkeit und 20°C.

Unter anderen zeigten in obigen Versuchen die Verbindungen Nr. 1.1, 1.6, 1.21, 2.2, 2.5, 2.9, 2.16, 3.1, 3.3, 3.7, 5.1, 5.3, 5.46, 5.48, 6.1, 7.1, 7.3 und 7.12 eine sehr gute systemische Wirkung. Gegenüber unbehandelten Kontrollpflanzen (100% Befall) bewirkten diese Verbindungen eine fast vollständige Unterdrückung des Pilzbefalls (0 bis 5%).

Beispiel B7: Wirkung gegen Plasmapora viticola auf Reben

a) Residual-protektive Wirkung

Im 4-5 Blattstadium wurden Rebensämlinge mit einer aus Spritzpulver des Wirkstoffes hergestellten Spritzbrühe (0,06% Aktivsubstanz) besprüht. Nach 24 Stunden wurden die behandelten Pflanzen mit einer Sporangiensuspension des Pilzes infiziert. Nach einer Inkubation während 6 Tagen bei 95-100% relativer Luftfeuchtigkeit und 20°C wurde der Pilzbefall beurteilt.

b) Residual-kurative Wirkung

Im 4-5 Blattstadium wurden Rebensämlinge mit einer Sporangiensuspension des Pilzes infiziert. Nach einer Inkubation während 24 Stunden in einer Feuchtkammer bei 95-100% relativer Luftfeuchtigkeit und 20°C wurden die infizierten Pflanzen getrocknet und mit einer aus Spritzpulver des Wirkstoffes hergestellten Spritzbrühe (0,06% Aktivsubstanz) besprüht. Nach dem Antrocknen des Spritzbelages wurden die behandelten Pflanzen wieder in die Feuchtkammer gebracht. Die Beurteilung des Pilzbefalls erfolgte 6 Tage nach der Infektion.

Verbindungen aus den Tabellen zeigten gegen Plasmopara viticola auf Regen eine sehr gute fungizide Wirkung, insbesondere die Wirkstoffe Nr. 1.1, 1.6, 1.7, 1.21, 2.2, 2.5, 2.9, 2.16, 3.1, 3.3, 5.1, 5.3, 5.4, 6.1, 6.2, 7.1, 7.12 und 7.19 bewirkten eine vollständige Unterdrückung des Pilzbefalls (0 bis 5 %).

Beispiel B8: Wirkung gegen Piricularia oryzae auf Reispflanzen

Residual-protektive Wirkung

Reispflanzen wurden nach zweiwöchiger Anzucht mit einer aus Spritzpulver des Wirkstoffes hergestellten Spritzbrühe (0,02% Aktivsubstanz) besprüht. Nach 48 Stunden wurden die behandelten Pflanzen mit einer Konidiensuspension des Pilzes infiziert. Nach 5 Tagen Inkubation bei 95-100% relativer Luftfeuchtigkeit und 24°C wurde der Pilzbefall beurteilt.

Reispflanzen, die mit einer Spritzbrühe behandelt worden sind, die als Aktivsubstanz eine Verbindung aus den Tabellen 2 oder 7, wie z.B. Nr. 7.1 und 7.19 enthielt, zeigten im Vergleich zu unbehandelten Kontrollpflanzen (100% Befall) weniger als 10% Pilzbefall.

Patentansprüche:

1. Verbindungen der Formel I

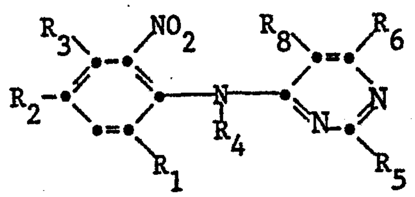

(I),

worin

$R_1$ für $NO_2$ oder $CF_3$ steht;

$R_2$ für $NO_2$ oder $CF_3$ steht;

$R_3$ Wasserstoff oder Halogen bedeutet;

$R_4$ Wasserstoff oder die Gruppe $-C(O)R_7$ steht; wobei

$R_7$ einen unsubstituierten oder substituierten Rest aus der Reihe $C_1$-$C_{12}$-Alkyl, $C_2$-$C_6$-Alkenyl, $C_3$-$C_7$-Cycloalkyl, Phenyl und Heterocyclyl repräsentiert;

$R_5$, $R_6$ und $R_8$ unabhängig voneinander für Halogen, Cyano, Rhodano, Nitro oder eine der Gruppen $-N(R_9)(R_{10})$, $-R_{11}$, $R_{12}$-O- oder $R_{13}$-S(O)$_n$ stehen, wobei $R_9$ und $R_{10}$ unabhängig voneinander Wasserstoff oder einen unsubstituierten oder substituierten Rest aus der Reihe $C_1$-$C_{12}$-Alkyl, $C_3$-$C_6$-Alkenyl und Phenyl darstellen oder zusammen mit dem Stickstoffatom gemeinsam einen gegebenenfalls substituierten heterocyclischen Ring bilden;

n eine der Zahlen 0, 1 oder 2 bedeutet;

$R_{11}$, $R_{12}$ und $R_{13}$ unabhängig voneinander für einen unsubstituierten oder substituierten Rest aus der Reihe $C_1$-$C_{12}$-Alkyl, $C_2$-$C_6$-Alkenyl, $C_3$-$C_6$-Alkinyl, $C_3$-$C_7$-Cycloalkyl, Phenyl oder Heterocyclyl stehen; darüberhinaus $R_5$ für Wasserstoff, $R_6$ für Wasserstoff oder zwei der Reste $R_5$, $R_6$ und $R_8$ für Wasserstoff stehen können.

2. Verbindungen der Formel I nach Anspruch 1, worin $R_1$ für $NO_2$ oder $CF_3$ steht; $R_2$ für $NO_2$ oder $CF_3$ steht; $R_3$ Wasserstoff oder Halogen

bedeutet; $R_4$ für Wasserstoff oder die Gruppe $-C(O)R_7$ steht; wobei $R_7$ unsubstituiertes oder durch Halogen, $C_1$-$C_3$-Alkoxy oder $C_1$-$C_3$-Alkylthio substituiertes $C_1$-$C_4$-Alkyl, unsubstituiertes oder durch Halogen substituiertes $C_2$-$C_4$-Alkenyl, $C_3$-$C_6$-Cycloalkyl, unsubstituiertes oder durch Halogen, Nitro oder $C_1$-$C_3$-Alkyl substituiertes Phenyl oder einen unsubstituierten oder durch Halogen, $C_1$-$C_3$-Alkyl oder Nitro substituierten, ungesättigten oder gesättigten, fünf- bis sechsgliedrigen heterocyclischen Ring mit ein bis drei gleichen oder verschiedenen Heteroatomen repräsentiert; $R_5$, $R_6$ und $R_8$ unabhängig voneinander für Halogen, Cyano, Rhodano, Nitro oder eine der Gruppen $-N(R_9)(R_{10})$, $-R_{11}$, $R_{12}$-O- oder $R_{13}$-$S(O)_n$ stehen, wobei $R_9$ und $R_{10}$ unabhängig voneinander für Wasserstoff, unsubstituiertes oder durch Hydroxy oder $C_1$-$C_4$-Alkoxy substituiertes $C_1$-$C_{12}$-Alkyl, $C_3$-$C_6$-Alkenyl, unsubstituiertes oder durch Halogen, Nitro, $C_1$-$C_4$-Alkyl oder $C_1$-$C_4$-Alkoxy substituiertes Phenyl stehen oder zusammen mit dem Stickstoffatom gemeinsam einen ungesättigten oder gesättigten, unsubstituierten oder durch Halogen, $C_1$-$C_4$-Alkyl oder $C_1$-$C_4$-Alkoxy substituierten, fünf- bis sechsgliedrigen heterocyclischen Ring bilden, der noch ein oder zwei zusätzliche Heteroatomen enthalten kann; n eine der Zahlen 0, 1 oder 2 bedeutet; $R_{11}$, $R_{12}$ und $R_{13}$ unabhängig voneinander für unsubstituiertes oder durch Halogen substituiertes $C_2$-$C_6$-Alkenyl; unsubstituiertes oder durch Halogen, Nitro, Cyano, Phenyl, Phenoxy, Phenylthio oder durch einen gesättigten oder ungesättigten, fünf- bis sechsgliedrigen heterocyclischen Ring mit ein bis drei Heteroatomen substituiertes $C_1$-$C_{12}$-Alkyl, wobei jeder cyclische Rest seinerseits unsubstituiert oder durch Halogen, Nitro, $C_1$-$C_4$-Alkyl, $C_1$-$C_4$-Haloalkyl oder $C_1$-$C_4$-Alkoxy substituiert ist; unsubstituiertes oder durch Halogen substituiertes $C_3$-$C_6$-Alkinyl; unsubstituiertes oder durch Halogen oder $C_1$-$C_3$-Alkyl substituiertes $C_3$-$C_7$-Cycloalkyl, unsubstituiertes oder durch Halogen, $C_1$-$C_4$-Haloalkyl, $C_1$-$C_4$-Alkyl oder $C_1$-$C_4$-Alkoxy substituiertes Phenyl oder für einen ungesättigten oder gesättigten, fünf- bis sechsgliedrigen unsubstituierten oder durch Halogen, Nitro, $C_1$-$C_4$-Alkyl, $C_1$-$C_4$-Haloalkyl oder $C_1$-$C_4$-Alkoxy substituierten heterocyclischen Ring mit ein bis drei

Heteroatomen stehen; und darüberhinaus $R_5$ für Wasserstoff, $R_6$ für Wasserstoff oder zwei der Reste $R_5$, $R_6$ und $R_8$ für Wasserstoff stehen können.

3. Verbindungen der Formel I nach Anspruch 2, worin $R_1$ für $NO_2$ oder $CF_3$ steht; $R_2$ für $NO_2$ oder $CF_3$ steht; $R_3$ Wasserstoff oder Halogen bedeutet; $R_4$ für Wasserstoff steht; $R_5$, $R_6$ und $R_8$ unabhängig voneinander für Halogen, Cyano, Rhodano, Nitro oder eine der Gruppen $-N(R_9)(R_{10})$, $-R_{11}$, $R_{12}-O-$ oder $R_{13}-S(O)_n$ stehen, wobei $R_9$ und $R_{10}$ unabhängig voneinander für Wasserstoff, unsubstituiertes oder durch Hydroxy oder $C_1$-$C_4$-Alkoxy substituiertes $C_1$-$C_{12}$-Alkyl, $C_3$-$C_6$-Alkenyl, unsubstituiertes oder durch Halogen, Nitro, $C_1$-$C_4$-Alkyl oder $C_1$-$C_4$-Alkoxy substituiertes Phenyl stehen oder zusammen mit dem Stickstoffatom gemeinsam einen ungesättigten oder gesättigten, unsubstituierten oder durch Halogen, $C_1$-$C_4$-Alkyl oder $C_1$-$C_4$-Alkoxy substituierten, fünf- bis sechsgliedrigen heterocyclischen Ring bilden, der noch ein oder zwei zusätzliche Heteroatome enthalten kann; n eine der Zahlen 0, 1 oder 2 bedeutet; $R_{11}$, $R_{12}$ und $R_{13}$ unabhängig voneinander für unsubstituiertes oder durch Halogen substituiertes $C_2$-$C_6$-Alkenyl; unsubstituiertes oder durch Halogen, Nitro, Cyano, Phenyl, Phenoxy, Phenylthio oder durch einen gesättigten oder ungesättigten, fünf- bis sechsgliedrigen heterocyclischen Ring mit ein bis drei Heteroatomen substituiertes $C_1$-$C_{12}$-Alkyl stehen, wobei jeder cyclische Rest seinerseits unsubstituiert oder durch Halogen, Nitro, $C_1$-$C_4$-Alkyl, $C_1$-$C_4$-Haloalkyl oder $C_1$-$C_4$-Alkoxy substituiert ist; unsubstituiertes oder durch Halogen substituiertes $C_3$-$C_6$-Alkinyl; unsubstituiertes oder durch Halogen oder $C_1$-$C_3$-Alkyl substituiertes $C_3$-$C_7$-Cycloalkyl, unsubstituiertes oder durch Halogen, $C_1$-$C_4$-Haloalkyl, $C_1$-$C_4$-Alkyl oder $C_1$-$C_4$-Alkoxy substituiertes Phenyl oder für einen ungesättigten oder gesättigten, fünf- bis sechsgliedrigen, unsubstituierten oder durch Halogen, Nitro, $C_1$-$C_4$-Alkyl, $C_1$-$C_4$-Haloalkyl oder $C_1$-$C_4$-Alkoxy substituierten heterocyclischen Ring mit

ein bis drei Heteroatomen stehen; und darüberhinaus $R_5$ für Wasserstoff, $R_6$ für Wasserstoff oder zwei der Reste $R_5$, $R_6$ und $R_8$ für Wasserstoff stehen können.

4. Verbindungen der Formel I nach Anspruch 3, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_5$ für Halogen, Cyano, Rhodano, Nitro oder eine der Gruppen $-N(R_9)(R_{10})$, $-R_{11}$, $R_{12}-O-$ oder $R_{13}-S(O)_n$ steht, wobei $R_9$ und $R_{10}$ unabhängig voneinander für Wasserstoff, $C_1$-$C_4$-Alkyl, $C_3$-$C_4$-Alkenyl, unsubstituiertes oder durch Halogen, Nitro, Methyl oder Methoxy substituiertes Phenyl stehen oder zusammen einen unsubstituierten oder durch Methyl substituierten Piperidinring bilden; n für die Zahl 2 steht; $R_{11}$, $R_{12}$ und $R_{13}$ unabhängig voneinander für $C_3$-$C_4$-Alkenyl, unsubstituierts oder durch Halogen, Nitro, Cyano, Phenyl, Phenoxy, Phenylthio oder durch einen ungesättigten oder gesättigten, fünf- oder sechsgliedrigen heterocyclischen Ring mit ein oder zwei Heteroatomen aus der Reihe Stickstoff, Sauerstoff und Schwefel substituiertes $C_1$-$C_{12}$-Alkyl, wobei jeder heterocyclische Rest seinerseits unsubstituiert oder durch Halogen, Nitro, Methyl, $CF_3$ oder Methoxy substituiert ist; für $C_3$-$C_4$-Alkinyl, $C_3$-$C_6$-Cycloalkyl, unsubstituiertes oder durch Halogen, Nitro, $C_1$-$C_2$-Alkyl, $CF_3$ oder $C_1$-$C_2$-Alkoxy substituiertes Phenyl stehen; und $R_6$ und $R_8$ Wasserstoff bedeuten.

5. Verbindungen der Formel I nach Anspruch 3, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_5$ für Wasserstoff steht; $R_6$ für Wasserstoff, Halogen, Cyano, Rhodano, Nitro oder eine der Gruppen $-N(R_9)(R_{10})$, $-R_{11}$, $R_{12}-O-$ oder $R_{13}-S(O)_n$ steht, wobei $R_9$ und $R_{10}$ unabhängig voneinander für Wasserstoff, $C_1$-$C_3$-Alkyl oder zusammen mit dem Stickstoffatom für Piperidin stehen; n für die Zahl 2 steht; $R_{11}$, $R_{12}$ und $R_{13}$ unabhängig voneinander für $C_3$-$C_4$-Alkenyl, unsubstituiertes oder durch Halogen, Nitro, Cyano, Phenyl, Phenoxy

oder Phenylthio substituiertes $C_1$-$C_4$-Alkyl, $C_3$-$C_6$-Cycloalkyl oder unsubstituiertes oder durch Halogen, $CF_3$, $C_1$-$C_2$-Alkyl oder $C_1$-$C_2$-Alkoxy substituiertes Phenyl stehen; und $R_8$ Wasserstoff bedeutet.

6. Verbindungen der Formel I nach Anspruch 3, worin $R_1$ für $NO_2$ oder $CF_3$ steht; $R_2$ für $NO_2$ oder $CF_3$ steht; $R_3$ Wasserstoff oder Chlor bedeutet; $R_4$, $R_5$ und $R_6$ für Wasserstoff stehen; und $R_8$ Halogen, Cyano, Rhodano, Nitro oder eine der Gruppen $-N(R_9)(R_{10})$, $-R_{11}$, $R_{12}$-O- oder $R_{13}$-S(O)$_n$ repräsentiert, wobei $R_9$ und $R_{10}$ unabhängig voneinander für Wasserstoff, $C_1$-$C_3$-Alkyl oder zusammen mit dem Stickstoffatom für Piperidin stehen; n für die Zahl 2 steht; und $R_{11}$, $R_{12}$ und $R_{13}$ unabhängig voneinander für $C_3$-$C_4$-Alkenyl, unsubstituiertes oder durch Halogen, Nitro, Cyano, Phenyl, Phenoxy oder Phenylthio substituiertes $C_1$-$C_4$-Alkyl, $C_3$-$C_6$-Cycloalkyl oder unsubstituiertes oder durch Halogen, $CF_3$, $C_1$-$C_2$-Alkyl oder $C_1$-$C_2$-Alkoxy substituiertes Phenyl stehen.

7. Verbindungen der Formel I nach Anspruch 3, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_5$ und $R_8$ unabhängig voneinander für Halogen, Cyano, Rhodano, Nitro oder eine der Gruppen $-N(R_9)(R_{10})$, $-R_{11}$, $R_{12}$-O- oder $R_{13}$-S(O)$_n$ steht, wobei $R_9$ und $R_{10}$ unabhängig voneinander für Wasserstoff, $C_1$-$C_4$-Alkyl, $C_3$-$C_4$-Alkenyl, unsubstituiertes oder durch Halogen, Nitro, Methyl oder Methoxy substituiertes Phenyl stehen oder zusammen einen unsubstituierten oder durch Methyl substituierten Ring ausgewählt aus der Reihe Piperidin, Tetrahydrofuryl und Morpholin bilden; n für die Zahl 2 steht; $R_{11}$, $R_{12}$ und $R_{13}$ unabhängig voneinander für $C_3$-$C_4$-Alkylen, unsubstituiertes oder durch Halogen, Nitro, Cyano, Phenyl, Phenoxy, Phenylthio oder durch einen ungesättigten oder gesättigten, fünf- oder sechsgliedrigen, heterocyclischen Ring mit ein oder zwei Heteroatomen aus der Reihe Stickstoff, Sauerstoff und Schwefel substituiertes $C_1$-$C_{12}$-Alkyl, wobei jeder heterocyclische Rest seinerseits unsubstituiert oder durch Halogen, Nitro, Methyl, $CF_3$ oder Methoxy substituiert ist; für $C_3$-$C_4$-Alkinyl,

$C_3$-$C_6$-Cycloalkyl, unsubstituiert oder durch Halogen, Nitro, $C_1$-$C_2$-Alkyl, $CF_3$ oder $C_1$-$C_2$-Alkoxy substituiertes Phenyl stehen; und $R_6$ Wasserstoff bedeutet.

8. Verbindungen der Formel I nach Anspruch 3, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_6$ und $R_8$ unabhängig voneinander für Halogen, Cyano, Rhodano, Nitro oder eine der Gruppen $-N(R_9)(R_{10})$, $-R_{11}$, $R_{12}$-O- oder $R_{13}$-$S(O)_n$ steht, wobei $R_9$ und $R_{10}$ unabhängig voneinander für Wasserstoff, $C_1$-$C_4$-Alkyl, $C_3$-$C_4$-Alkenyl, unsubstituiertes oder durch Halogen, Nitro, Methyl oder Methoxy substituiertes Phenyl stehen oder zusammen einen unsubstituierten oder durch Methyl substituierten Piperidinring bilden; n für die Zahl 2 steht; $R_{11}$, $R_{12}$ und $R_{13}$ unabhängig voneinander für $C_3$-$C_4$-Alkenyl, unsubstituiertes oder durch Halogen, Nitro, Cyano, Phenyl, Phenoxy, Phenylthio oder durch einen ungesättigten oder gesättigten, fünf- oder sechsgliedrigen heterocyclischen Ring mit ein oder zwei Heteroatomen aus der Reihe Stickstoff, Sauerstoff und Schwefel substituiertes $C_1$-$C_4$-Alkyl, wobei jeder heterocyclische Rest seinerseits unsubstituiert oder durch Halogen, Nitro, Methyl, $CF_3$ oder Methoxy substituiert ist; für $C_3$-$C_4$-Alkinyl, $C_3$-$C_6$-Cycloalkyl, unsubstituiert oder durch Halogen, Nitro, $C_1$-$C_2$-Alkyl, $CF_3$ oder $C_1$-$C_2$-Alkoxy substituiertes Phenyl stehen; und $R_5$ Wasserstoff bedeutet.

9. Eine Verbindung der Formel I, ausgewählt aus der Reihe:

N-(3'-Chlor-2',6'-dinitro-4'-trifluormethylphenyl)-4-amino-6-chlor-pyrimidin (Nr. 2.2);

N-(3'-Chlor-2',6'-dinitro-4'-trifluormethylphenyl)-4-amino-6-methylmercapto-pyrimidin (Nr. 2.5);

N-(3'-Chlor-2',6'-dinitro-4'-trifluormethylphenyl)-4-maino-6-methoxy-pyrimidin (Nr. 2.4);

N-(3'-Chlor-2',6'-dinitro-4'-trifluormethylphenyl)-4-amino-6-(2,2,2-trifluorethoxy)-pyrimidin (Nr. 2.6);

N-(3'-Chlor-2',6'-dinitro-4'-trifluormethylphenyl)-4-amino-2-methoxy-5-chlor-pyrimidin (Nr. 5.1);

N-(3'-Chlor-2',6'-dinitro-4'-trifluormethylphenyl)-4-amino-2,5-dichlorpyrimidin (Nr. 5.3);

N-(3'-Chlor-2',6'-dinitro-4'-trifluormethylphenyl)-4-amino-2-chlor-5-fluor-pyrimidin (Nr. 5.4);

N-(3'-Chlor-2',6'-dinitro-4'-trifluormethylphenyl)-4-amino-2-methoxy-5-fluor-pyrimidin (Nr. 5.6);

N-(3'-Chlor-2',6'-dinitro-4'-trifluormethylphenyl)-4-amino-5-nitro-6-methoxy-pyrimidin (Nr. 6.1);

N-(3'-Chlor-2',6'-dinitro-4'-trifluormethylphenyl)-4-amino-5-nitro-6-amino-pyrimidin (Nr. 6.2);

N-(3'-Chlor-2',6'-dinitro-4'-trifluormethylphenyl)-4-amino-2-methyl-mercapto-5-nitro-6-methoxy-pyrimidin (Nr. 7.1);

N-(3'-Chlor-2',6'-dinitro-4'-trifluormethylphenyl)-4-amino-2-methyl-5-ethyl-6-chlor-pyrimidin (Nr. 7.3);

10. Verfahren zur Herstellung von Verbindungen der Formel I, dadurch gekennzeichnet, dass man eine Verbindung der Formel II

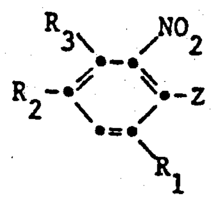

(II)

in Gegenwart einer Base mit einem Pyrimidin-Derivat der Formel III

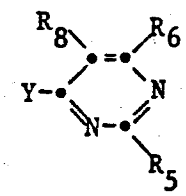

(III)

zu einer Verbindung der Formel I'

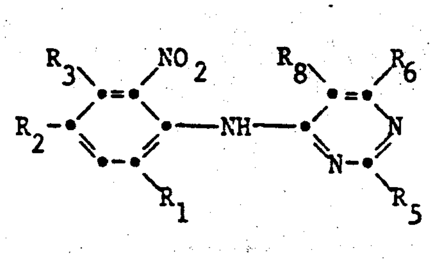

(I')

umsetzt, und letztere zur Herstellung N-acylierter Derivate mit einem reaktionsfähigen Derivat der Carbonsäure IV

$R_4COOH$ (IV)

N-acyliert, wobei die Substituenten $R_1$ bis $R_8$ die unter Formel I angegebenen Bedeutungen haben und Z und Y für $NH_2$ oder Halogen stehen, wobei in dem Fall, in dem Z für Halogen steht, Y $NH_2$ bedeutet und in dem Fall in dem Z für $NH_2$ steht, Y Halogen bedeutet.

11. Mittel zur Bekämpfung oder Verhütung eines Befalls durch Schädlinge, dadurch gekennzeichnet, dass es neben üblichen Zuschlagstoffen als mindestens eine aktive Komponente eine Verbindung der Formel I enthält.

12. Verfahren zur Bekämpfung oder Verhügung eines Befalls von Kulturpflanzen durch phytopathogene Schädlinge, dadurch gekennzeichnet, dass man eine Verbindung der Formel I auf die Pflanze oder deren Standort appliziert.

Patentansprüche für Oesterreich

1. Verfahren zur Herstellung von Verbindungen der Formel I

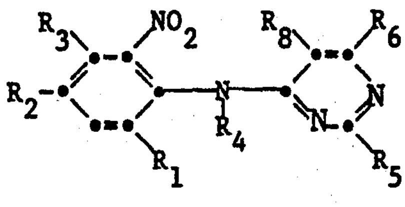

(I),

worin

$R_1$ für $NO_2$ oder $CF_3$ steht;

$R_2$ für $NO_2$ oder $CF_3$ steht;

$R_3$ Wasserstoff oder Halogen bedeutet;

$R_4$ Wasserstoff oder die Gruppe $-C(O)R_7$ steht; wobei

$R_7$ einen unsubstituierten oder substituierten Rest aus der Reihe $C_1$-$C_{12}$-Alkyl, $C_2$-$C_6$-Alkenyl, $C_3$-$C_7$-Cycloalkyl, Phenyl und Heterocyclyl repräsentiert;

$R_5$, $R_6$ und $R_8$ unabhängig voneinander für Halogen, Cyano, Rhodano, Nitro oder eine der Gruppen $-N(R_9)(R_{10})$, $-R_{11}$, $R_{12}$-O- oder $R_{13}$-$S(O)_n$ stehen, wobei $R_9$ und $R_{10}$ unabhängig voneinander Wasserstoff oder einen unsubstituierten oder substituierten Rest aus der Reihe $C_1$-$C_{12}$-Alkyl, $C_3$-$C_6$-Alkenyl und Phenyl darstellen oder zusammen mit dem Stickstoffatom gemeinsam einen gegebenenfalls substituierten heterocyclischen Ring bilden;

n eine der Zahlen 0, 1 oder 2 bedeutet;

$R_{11}$, $R_{12}$ und $R_{13}$ unabhängig voneinander für einen unsubstituierten oder substituierten Rest aus der Reihe $C_1$-$C_{12}$-Alkyl, $C_2$-$C_6$-Alkenyl, $C_3$-$C_6$-Alkinyl, $C_3$-$C_7$-Cycloalkyl, Phenyl oder Heterocyclyl stehen; darüberhinaus $R_5$ für Wasserstoff, $R_6$ für Wasserstoff oder zwei der Reste $R_5$, $R_6$ und $R_8$ für Wasserstoff stehen können, dadurch gekennzeichnet, dass man eine Verbindung der Formel II

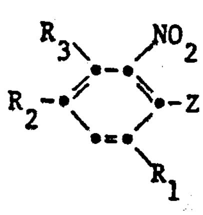

(II)

in Gegenwart einer Base mit einem Pyrimidin-Derivat der Formel III

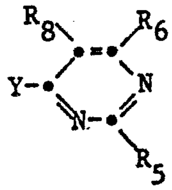

(III)

zu einer Verbindung der Formel I'

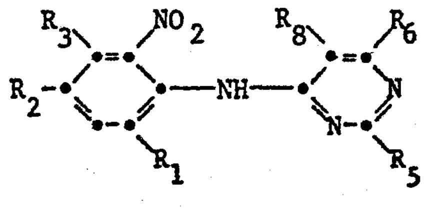

(I')

umsetzt, und letztere zur Herstellung N-acylierter Derivate mit einem reaktionsfähigen Derivat der Carbonsäure IV

$R_4COOH$ (IV)

N-acyliert, wobei die Substituenten $R_1$ bis $R_8$ die unter Formel I angegebenen Bedeutungen haben und Z und Y für $NH_2$ oder Halogen stehen, wobei in dem Fall, in dem Z für Halogen steht, Y $NH_2$ bedeutet und in dem Fall in dem Z für $NH_2$ steht, Y Halogen bedeutet.

2. Verfahren nach Anspruch 1, zur Herstellung von Verbindungen der Formel I, worin $R_1$ für $NO_2$ oder $CF_3$ steht; $R_2$ für $NO_2$ oder $CF_3$ steht; $R_3$ Wasserstoff oder Halogen bedeutet; $R_4$ für Wasserstoff oder die Gruppe $-C(O)R_7$ steht; wobei $R_7$ unsubstituiertes oder durch Halogen, $C_1$-$C_3$-Alkoxy oder $C_1$-$C_3$-Alkylthio substituiertes $C_1$-$C_4$-Alkyl, unsubstituiertes oder durch Halogen substituiertes $C_2$-$C_4$-Alkenyl, $C_3$-$C_6$-Cycloalkyl, unsubstituiertes oder durch Halogen, Nitro oder $C_1$-$C_3$-Alkyl substituiertes Phenyl oder einen unsubstituierten oder durch Halogen, $C_1$-$C_3$-Alkyl oder Nitro substituierten, ungesättigten oder gesättigten, fünf-

bis sechsgliedrigen heterocyclischen Ring mit ein bis drei gleichen oder verschiedenen Heteroatomen repräsentiert; $R_5$, $R_6$ und $R_8$ unabhängig voneinander für Halogen, Cyano, Rhodano, Nitro oder eine der Gruppen $-N(R_9)(R_{10})$, $-R_{11}$, $R_{12}-O-$ oder $R_{13}-S(O)_n$ stehen, wobei $R_9$ und $R_{10}$ unabhängig voneinander für Wasserstoff, unsubstituiertes oder durch Hydroxy oder $C_1-C_4$-Alkoxy substituiertes $C_1-C_{12}$-Alkyl, $C_3-C_6$-Alkenyl, unsubstituiertes oder durch Halogen, Nitro, $C_1-C_4$-Alkyl oder $C_1-C_4$-Alkoxy substituiertes Phenyl stehen oder zusammen mit dem Stickstoffatom gemeinsam einen ungesättigten oder gesättigten, unsubstituierten oder durch Halogen, $C_1-C_4$-Alkyl oder $C_1-C_4$-Alkoxy substituierten, fünf- bis sechsgliedrigen heterocyclischen Ring bilden, der noch ein oder zwei zusätzliche Heteroatomen enthalten kann; n eine der Zahlen 0, 1 oder 2 bedeutet; $R_{11}$, $R_{12}$ und $R_{13}$ unabhängig voneinander für unsubstituiertes oder durch Halogen substituiertes $C_2-C_6$-Alkenyl; unsubstituiertes oder durch Halogen, Nitro, Cyano, Phenyl, Phenoxy, Phenylthio oder durch einen gesättigten oder ungesättigten, fünf- bis sechsgliedrigen heterocyclischen Ring mit ein bis drei Heteroatomen substituiertes $C_1-C_{12}$-Alkyl, wobei jeder cyclische Rest seinerseits unsubstituiert oder durch Halogen, Nitro, $C_1-C_4$-Alkyl, $C_1-C_4$-Haloalkyl oder $C_1-C_4$-Alkoxy substituiert ist; unsubstituiertes oder durch Halogen substituiertes $C_3-C_6$-Alkinyl; unsubstituiertes oder durch Halogen oder $C_1-C_3$-Alkyl substituiertes $C_3-C_7$-Cycloalkyl, unsubstituiertes oder durch Halogen, $C_1-C_4$-Haloalkyl, $C_1-C_4$-Alkyl oder $C_1-C_4$-Alkoxy substituiertes Phenyl oder für einen ungesättigten oder gesättigten, fünf- bis sechsgliedrigen unsubstituierten oder durch Halogen, Nitro, $C_1-C_4$-Alkyl, $C_1-C_4$-Haloalkyl oder $C_1-C_4$-Alkoxy substituierten heterocyclischen Ring mit ein bis drei Heteroatomen stehen; und darüberhinaus $R_5$ für Wasserstoff, $R_6$ für Wasserstoff oder zwei der Reste $R_5$, $R_6$ und $R_8$ für Wasserstoff stehen können.

3. Verfahren nach Anspruch 2, zur Herstellung von Verbindungen der Formel I, worin $R_1$ für $NO_2$ oder $CF_3$ steht. $R_2$ für $NO_2$ oder $CF_3$

steht; $R_3$ Wasserstoff oder Halogen bedeutet; $R_4$ für Wasserstoff steht; $R_5$, $R_6$ und $R_8$ unabhängig voneinander für Halogen, Cyano, Rhodano, Nitro oder eine der Gruppen $-N(R_9)(R_{10})$, $-R_{11}$, $R_{12}$ -O- oder $R_{13}$ $-S(O)_n$ stehen, wobei $R_9$ und $R_{10}$ unabhängig voneinander für Wasserstoff, unsubstituiertes oder durch Hydroxy oder $C_1$-$C_4$-Alkoxy substituiertes $C_1$-$C_{12}$-Alkyl, $C_3$-$C_6$-Alkenyl, unsubstituiertes oder durch Halogen, Nitro, $C_1$-$C_4$-Alkyl oder $C_1$-$C_4$-Alkoxy substituiertes Phenyl stehen oder zusammen mit dem Stickstoffatom gemeinsam einen ungesättigten oder gesättigten, unsubstituierten oder durch Halogen, $C_1$-$C_4$-Alkyl oder $C_1$-$C_4$-Alkoxy substituierten, fünf- bis sechsgliedrigen heterocyclischen Ring bilden, der noch ein oder zwei zusätzliche Heteroatome enthalten kann;
n eine der Zahlen 0, 1 oder 2 bedeutet; $R_{11}$, $R_{12}$ und $R_{13}$ unabhängig voneinander für unsubstituiertes oder durch Halogen substituiertes $C_2$-$C_6$-Alkenyl; unsubstituiertes oder durch Halogen, Nitro, Cyano, Phenyl, Phenoxy, Phenylthio oder durch einen gesättigten oder ungesättigten, fünf- bis sechsgliedrigen heterocyclischen Ring mit ein bis drei Heteroatomen substituiertes $C_1$-$C_{12}$-Alkyl, wobei jeder cyclische Rest seinerseits unsubstituiert oder durch Halogen, Nitro, $C_1$-$C_4$-Alkyl, $C_1$-$C_4$-Haloalkyl oder $C_1$-$C_4$-Alkoxy substituiert ist; unsubstituiertes oder durch Halogen substituiertes $C_3$-$C_6$-Alkinyl; unsubstituiertes oder durch Halogen oder $C_1$-$C_3$-Alkyl substituiertes $C_3$-$C_7$-Cycloalkyl, unsubstituiertes oder durch Halogen, $C_1$-$C_4$-Haloalkyl, $C_1$-$C_4$-Alkyl oder $C_1$-$C_4$-Alkoxy substituiertes Phenyl oder für einen ungesättigten oder gesättigten, fünf- bis sechsgliedrigen, unsubstituierten oder durch Halogen, Nitro, $C_1$-$C_4$-Alkyl, $C_1$-$C_4$-Haloalkyl oder $C_1$-$C_4$-Alkoxy substituierten heterocyclischen Ring mit ein bis drei Heteroatomen stehen; und darüberhinaus $R_5$ für Wasserstoff, $R_6$ für Wasserstoff oder zwei der Reste $R_5$, $R_6$ und $R_8$ für Wasserstoff stehen können.

4. Verfahren nach Anspruch 3, zur Herstellung von Verbindungen der Formel I, worin $R_1$ für $NO_2$ oder $CF_3$ steht; $R_3$ Wasserstoff oder

Chlor bedeutet; $R_4$ für Wasserstoff steht; $R_5$ für Halogen, Cyano, Rhodano, Nitro oder eine der Gruppen $-N(R_9)(R_{10})$, $-R_{11}$, $R_{12}$ -O- oder $R_{13}$ $-S(O)_n$ steht, wobei $R_9$ und $R_{10}$ unabhängig voneinander für Wasserstoff, $C_1$-$C_4$-Alkyl, $C_3$-$C_4$-Alkenyl, unsubstituiertes oder durch Halogen, Nitro, Methyl oder Methoxy substituiertes Phenyl stehen oder zusammen einen unsubstituierten oder durch Methyl substituierten Piperidinring bilden; n für die Zahl 2 steht; $R_{11}$, $R_{12}$ und $R_{13}$ unabhängig voneinander für $C_3$-$C_4$-Alkenyl, unsubstituierts oder durch Halogen, Nitro, Cyano, Phenyl, Phenoxy, Phenylthio oder durch einen ungesättigten oder gesättigten, fünf- oder sechsgliedrigen heterocyclischen Ring mit ein oder zwei Heteroatomen aus der Reihe Stickstoff, Sauerstoff und Schwefel substituiertes $C_1$-$C_{12}$-Alkyl, wobei jeder heterocyclische Rest seinerseits unsubstituiert oder durch Halogen, Nitro, Methyl, $CF_3$ oder Methoxy substituiert ist; für $C_3$-$C_4$-Alkinyl, $C_3$-$C_6$-Cycloalkyl, unsubstituiertes oder durch Halogen, Nitro, $C_1$-$C_2$-Alkyl, $CF_3$ oder $C_1$-$C_2$-Alkoxy substituiertes Phenyl stehen; und $R_6$ und $R_8$ Wasserstoff bedeuten.

5. Verfahren nach Anspruch 3, zur Herstellung von Verbindungen der Formel I, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_5$ für Wasserstoff steht; $R_6$ für Wasserstoff, Halogen, Cyano, Rhodano, Nitro oder eine der Gruppen $-N(R_9)(R_{10})$, $-R_{11}$, $R_{12}$ -O- oder $R_{13}$ $-S(O)_n$ steht, wobei $R_9$ und $R_{10}$ unabhängig voneinander für Wasserstoff, $C_1$-$C_3$-Alkyl oder zusammen mit dem Stickstoffatom für Piperidin stehen; n für die Zahl 2 steht; $R_{11}$, $R_{12}$ und $R_{13}$ unabhängig voneinander für $C_3$-$C_4$-Alkenyl unsubstituiertes oder durch Halogen, Nitro, Cyano, Phenyl, Phenoxy oder Phenylthio substituiertes $C_1$-$C_4$-Alkyl, $C_3$-$C_6$-Cycloalkyl oder unsubstituiertes oder durch Halogen, $CF_3$, $C_1$-$C_2$-Alkyl oder $C_1$-$C_2$-Alkoxy substituiertes Phenyl stehen; und $R_8$ Wasserstoff bedeutet.

6. Verfahren nach Anspruch 3, zur Herstellung von Verbindungen der Formel I, worin $R_1$ für $NO_2$ oder $CF_3$ steht; $R_2$ für $NO_2$ oder $CF_3$ steht; $R_3$ Wasserstoff oder Chlor bedeutet; $R_4$, $R_5$ und $R_6$ für Wasserstoff

stehen; und $R_8$ Halogen, Cyano, Rhodano, Nitro oder eine der Gruppen $-N(R_9)(R_{10})$, $-R_{11}$, $R_{12}$-O- oder $R_{13}$-S(O)$_n$ repräsentiert, wobei $R_9$ und $R_{10}$ unabhängig voneinander für Wasserstoff, $C_1$-$C_3$-Alkyl oder zusammen mit dem Stickstoffatom für Piperidin stehen; n für die Zahl 2 steht; und $R_{11}$,$R_{12}$ und $R_{13}$ unabhängig voneinander für $C_3$-$C_4$-Alkenyl, unsubstituiertes oder durch Halogen, Nitro, Cyano, Phenyl, Phenoxy oder Phenylthio substituiertes $C_1$-$C_4$-Alkyl, $C_3$-$C_6$-Cycloalkyl oder unsubstituiertes oder durch Halogen, $CF_3$, $C_1$-$C_2$-Alkyl oder $C_1$-$C_2$-Alkoxy substituiertes Phenyl stehen.

7. Verfahren nach Anspruch 3, zur Herstellung von Verbindungen der Formel I, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_5$ und $R_8$ unabhängig voneinander für Halogen, Cyano, Rhodano, Nitro oder eine der Gruppen $-N(R_9)(R_{10})$, $R_{11}$, $R_{12}$ -O- oder $R_{13}$ -S(O)$_n$ steht, wobei $R_9$ und $R_{10}$ unabhängig voneinander für Wasserstoff, $C_1$-$C_4$-Alkyl, $C_3$-$C_4$-Alkenyl, unsubstituiertes oder durch Halogen, Nitro, Methyl oder Methoxy substituiertes Phenyl stehen oder zusammen einen unsubstituierten oder durch Methyl substituierten Ring ausgewählt aus der Reihe Piperidin, Tetrahydrofuryl und Morpholin bilden; n für die Zahl 2 steht; $R_{11}$, $R_{12}$ und $R_{13}$ unabhängig voneinander für $C_3$-$C_4$-Alkenyl, unsubstituiertes oder durch Halogen, Nitro, Cyano, Phenyl, Phenoxy, Phenylthio oder durch einen ungesättigten oder gesättigten, fünf- oder sechsgliedrigen, heterocyclischen Ring mit ein oder zwei Heteroatomen aus der Reihe Stickstoff, Sauerstoff und Schwefel substituiertes $C_1$-$C_{12}$-Alkyl, wobei jeder heterocyclische Rest seinerseits unsubstituiert oder durch Halogen, Nitro, Methyl, $CF_3$ oder Methoxy substituiert ist; für $C_3$-$C_4$-Alkinyl, $C_3$-$C_6$-Cycloalkyl, unsubstituiert oder durch Halogen, Nitro, $C_1$-$C_2$-Alkyl, $CF_3$ oder $C_1$-$C_2$-Alkoxy substituiertes Phenyl stehen; und $R_6$ Wasserstoff bedeutet.

8. Verfahren nach Anspruch 3, zur Herstellung von Verbindungen der Formel I, worin $R_1$ für $NO_2$ oder $CF_3$ steht; $R_2$ für $NO_2$ oder $CF_3$ steht; $R_3$ Wasserstoff oder Chlor bedeutet; $R_4$ für Wasserstoff steht; $R_6$ und

$R_8$ unabhängig voneinander für Halogen, Cyano, Rhodano, Nitro oder eine der Gruppen $-N(R_9)(R_{10}9$, $-R_{11}$, $R_{12}$, -O- oder $R_{13}$ $-S(O)_n$ steht, wobei $R_9$ und $R_{10}$ unabhängig voneinander für Wasserstoff, $C_1$-$C_4$-Alkyl, $C_3$-$C_4$-Alkenyl, unsubstituiertes oder durch Halogen, Nitro, Methyl oder Methoxy substituiertes Phenyl stehen oder zusammen einen unsubstituierten oder durch Methyl substituierten Piperidinring bilden; n für die Zahl 2 steht; $R_{11}$, $R_{12}$ und $R_{13}$ unabhängig voneinander für $C_3$-$C_4$-Alkenyl, unsubstituiertes oder durch Halogen, Nitro, Cyano, Phenyl, Phenoxy, Phenylthio oder durch einen ungesättigten oder gesättigten, fünf- oder sechsgliedrigen heterocyclischen Ring mit ein oder zwei Heteroatomen aus der Reihe Stickstoff, Sauerstoff und Schwefel substituiertes $C_1$-$C_4$-Alkyl, wobei jeder heterocyclische Rest seinerseits unsubstituiert oder durch Halogen, Nitro, Methyl, $CF_3$ oder Methoxy substituiert ist; für $C_3$-$C_4$-Alkinyl, $C_3$-$C_6$-Cycloalkyl, unsubstituiert oder durch Halogen, Nitro, $C_1$-$C_2$-Alkyl, $CF_3$ oder $C_1$-$C_2$-Alkoxy substituiertes Phenyl stehen; und $R_5$ Wasserstoff bedeutet.

9. Verfahren nach Anspruch 1, zur Herstellung einer Verbindung der Formel I, ausgewählt aus der Reihe:

N-(3'-Chlor-2',6'-dinitro-4'-trifluormethylphenyl)-4-amino-6-chlor-pyrimidin (Nr. 2.2);

N-(3'-Chlor-2',6'-dinitro-4'-trifluormethylphenyl)-4-amino-6-methylmercapto-pyrimidin (Nr. 2.5);

N-(3'-Chlor-2',6'-dinitro-4'-trifluormethylphenyl)-4-maino-6-methoxy-pyrimidin (Nr. 2.4);

N-(3'-Chlor-2',6'-dinitro-4'-trifluormethylphenyl)-4-amino-6-(2,2,2-trifluorethoxy)-pyrimidin (Nr. 2.6);

N-(3'-Chlor-2',6'-dinitro-4'-trifluormethylphenyl)-4-amino-2-methoxy-5-chlor-pyrimidin (Nr. 5.1);

N-(3'-Chlor-2',6'-dinitro-4'-trifluormethylphenyl)-4-amino-2,5-dichlorpyrimidin (Nr. 5.3);

N-(3'-Chlor-2',6'-dinitro-4'-trifluormethylphenyl)-4-amino-2-chlor-5-fluor-pyrimidin (Nr. 5.4);

N-(3'-Chlor-2',6'-dinitro-4'-trifluormethylphenyl)-4-amino-2-methoxy-5-fluor-pyrimidin (Nr. 5.6);

N-(3'-Chlor-2',6'-dinitro-4'-trifluormethylphenyl)-4-amino-5-nitro-6-methoxy-pyrimidin (Nr. 6.1);

N-(3'-Chlor-2',6'-dinitro-4'-trifluormethylphenyl)-4-amino-5-nitro-6-amino-pyrimidin (Nr. 6.2);

N-(3'-Chlor-2',6'-dinitro-4'-trifluormethylphenyl)-4-amino-2-methylmercapto-5-nitro-6-methoxy-pyrimidin (Nr. 7.1);

N-(3'-Chlor-2',6'-dinitro-4'-trifluormethylphenyl)-4-amino-2-methyl-5-ethyl-6-chlor-pyrimidin (Nr. 7.3);

10. Mittel zur Bekämpfung oder Verhütung eines Befalls durch Schädlinge, dadurch gekennzeichnet, dass es neben üblichen Zuschlagstoffen als mindestens eine aktive Komponente eine Verbindung der Formel I gemäss Anspruch 1 enthält.

11. Mittel gemäss Anspruch 10 dadurch gekennzeichnet, dass es als mindestens eine aktive Komponente eine Verbindung der Formel I gemäss Anspruch 2 enthält.

12. Mittel zur Bekämpfung von Mikroorganismen gemäss Anspruch 10 dadurch gekennzeichnet, dass es als mindestens eine aktive Komponente eine Verbindung der Formel I gemäss einem der Ansprüche 3 bis 9 enthält.

13. Mittel nach Anspruch 12 dadurch gekennzeichnet, dass es 0,1 bis 99% eines Wirkstoffs der Formel I, 99,9 bis 1% eines festen oder flüssigen Zusatzstoffes und 0 bis 25% eines Tensides enthält.

14. Mittel nach Anspruch 13 dadurch gekennzeichnet, dass es 0,1 bis 95% eines Wirkstoffs der Formel I, 99,8 bis 5% eines festen oder flüssigen Zusatzstoffes und 0,1 bis 25% eines Tensids enthält.

15. Verfahren zur Herstellung eines wie in Anspruch 10 beanspruchten agrochemischen Mittel, dadurch gekennzeichnet, dass man mindestens eine gemäss Anspruch 1 definierte Verbindung der Formel I mit geeigneten festen oder flüssigen Zusatzstoffen und Tensiden innig vermischt.

16. Verfahren zur Bekämpfung oder Verhütung eines Befalls von Kulturpflanzen durch phytopathogene Schädlinge, dadurch gekennzeichnet, dass man eine gemäss Anspruch 1 definierte Verbindung der Formel I auf die Pflanze oder deren Standort appliziert.

17. Verwendung von Verbindungen der Formel I gemäss Anspruch 1 zur Bekämpfung und/oder präventiven Verhütung eines Befalls von Mikroorganismen.

FO 7.5/HL/cw*/we*

0139613

Europäisches Patentamt

# EUROPÄISCHER RECHERCHENBERICHT

Nummer der Anmeldung: EP 84810417.0

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 4) |
|---|---|---|---|
| A | EP - A1 - 0 067 630 (SANKYO) * Ansprüche 1,8 * -- | 1,10 | C 07 D 239/47<br>C 07 D 239/42<br>C 07 D 239/46<br>C 07 D 239/48<br>C 07 D 239/28<br>A 01 N 43/54 |
| A | EP - A1 - 0 057 440 (SANKYO) * Ansprüche 1,22; Zusammenfassung * ---- | 1,10, 11,12 | |

RECHERCHIERTE SACHGEBIETE (Int. Cl.4)

C 07 D 239/00

Der vorliegende Recherchenbericht wurde für alle Patentansprüche erstellt.

| Recherchenort | Abschlußdatum der Recherche | Prüfer |
|---|---|---|
| WIEN | 04-12-1984 | HOCHHÄUSER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82